# EUROPÄISCHE PATENTSCHRIFT

(11) **EP 3 390 339 B1**
(45) Veröffentlichungstag und Bekanntmachung des Hinweises auf die Patenterteilung: **08.01.2020**
(21) Anmeldenummer: 16801531.1
(22) Anmeldetag: 28.11.2016
(51) Int. Cl.: C07C 67/04, C07C 67/54, C07C 67/62, B01D 3/14

(54) **HERSTELLUNG VON TERT-BUTYLESTERN ETHYLENISCH UNGESÄTTIGTER CARBONSÄUREN**
PRODUCTION OF TERT-BUTYL ESTERS OF ETHYLENICALLY UNSATURATED CARBOXYLIC ACIDS
PRODUCTION D'ESTERS TERTIOBUTYLIQUES D'ACIDES CARBOXYLIQUES ETHYLÉNIQUEMENT INSATURÉS

(30) Priorität: 15.12.2015 DE 102015121860; 15.12.2015 US 201562267331 P
(43) Veröffentlichungstag der Anmeldung: 24.10.2018
(73) Patentinhaber: BASF SE, 67056 Ludwigshafen am Rhein (DE)
(72) Erfinder: HORSTMANN, Catharina, 67056 Ludwigshafen (DE); HECHLER, Claus, 67056 Ludwigshafen (DE); GRACKIEWICZ, Gregor, 67056 Ludwigshafen (DE); SCHALL, Bernd, 67056 Ludwigshafen (DE)
(74) Vertreter: Reitstötter Kinzebach
(86) Internationale Anmeldenummer: PCT/EP2016/079025
(87) Internationale Veröffentlichungsnummer: WO 2017/102297

(56) Entgegenhaltungen:
- WO-A1-02/10109
- DE-A1-102008 002 923

## Beschreibung

Die vorliegende Erfindung betrifft ein kontinuierliches Verfahren zur Herstellung des tert-Butylesters einer ethylenisch ungesättigten Carbonsäure durch Umsetzung der Carbonsäure mit Isobuten.

Die tert-Butylester ethylenisch ungesättigter Carbonsäuren finden vielfältige Anwendung. Beispielsweise sind (Meth)acrylsäure-tert-butylester wichtige Ausgangsstoffe zur Herstellung von Polymerisaten, die u. a. als Bestandteil von Anstrichmitteln, Klebstoffen oder Lackharzen Anwendung finden. Die Herstellung solcher tert-Butylester erfolgt im Allgemeinen durch säurekatalysierte Addition einer Carbonsäure an Isobuten (Houben-Weyl, Methoden der Organischen Chemie, Bd. 8, 1952, S. 534; US 3,031,495 und US 3,082,246). Als Katalysatoren verwendet man dabei im Reaktionsgemisch lösliche Säuren, z. B. Mineralsäuren oder Alkyl- bzw. Arylsulfonsäuren (DE-A-12 49 857, US 3,087,962, US 3,088,969) oder unlösliche Katalysatoren wie saure Austauscherharze (US 3,037,052, US 3,031,495, DE-A-31 05 399, EP-A-268 999).

Die WO 02/10109 A1 beschreibt ein Verfahren zur kontinuierlichen Herstellung des tert-Butylesters einer aliphatischen C₁-C₄-Carbonsäure durch Umsetzung der Carbonsäure mit Isobuten in flüssiger Phase in Gegenwart eines sauren Katalysators in einem Reaktor, wobei man den Ester destillativ aus dem Reaktionsgemisch gewinnt, das man nach Entfernen des nicht umgesetzten Isobutens und der Leichtsieder erhält.

Die WO 02/10110 A2 beschreibt ein Verfahren zur Herstellung eines tert-Alkyl-(meth)acrylats durch Umsetzung von (Meth)acrylsäure mit einem Olefin in homogener Phase in Gegenwart eines sauren Katalysators und destillativer Abtrennung des tert-Alkyl(meth)acrylats aus dem Reaktionsgemisch, das man nach Entfernen des nicht umgesetzten Isobutens und der Leichtsieder erhält.

Ethylenisch ungesättigte Carbonsäuren und ihre Ester können vor allem bei höherer Temperatur eine starke Polymerisationsneigung aufweisen. Insbesondere bei Destillationen sind diese Verbindungen im Allgemeinen Temperaturen ausgesetzt, die leicht eine unerwünschte Polymerisation auslösen können. Dies hat eine Verschmutzung der Apparaturen, das Verstopfen von Leitungen und Pumpen und die Belegung von Kolonnenböden und Wärmeaustauscherflächen zur Folge. Das Reinigen der Anlagen ist ein aufwendiger, teurer und umweltbelastender Vorgang, und die Verfügbarkeit der Anlagen wird dadurch stark reduziert. Außerdem können unkontrollierte radikalische Polymerisationen ein Sicherheitsrisiko darstellen.

Der vorliegenden Erfindung liegt daher die Aufgabe zugrunde, ein Verfahren zur kontinuierlichen Herstellung des tert-Butylesters einer ethylenisch ungesättigten Carbonsäure bereitzustellen, bei dem die Trennung des tert-Butylesters von nicht umgesetzter Carbonsäure unter möglichst geringer Polymerisation sowohl des tert-Butylesters als auch der Carbonsäure verläuft.

Die WO 2011/110257 A2 beschreibt ein Verfahren zum Gewinnen einer leicht polymerisierbaren Verbindung wie (Meth)acrylsäure aus einem flüssigen Stoffgemisch durch Destillation in einer Destillationsvorrichtung, wobei im unteren Teil der Destillationsvorrichtung ein Rückführstrom abgezogen wird, welcher als überhitzter, flüssiger Rückführstrom in die Destillationsvorrichtung entspannt wird. Die leicht polymerisierbare Verbindung wird so von beispielsweise Lösungsmitteln abgetrennt.

Die WO 2011/110257 A2 beschreibt nicht die Abtrennung eines ethylenisch ungesättigten Esters von nicht umgesetzter ethylenisch ungesättigter Carbonsäure. Diese destillative Trennung von zwei leicht polymerisierbaren Verbindungen ist anspruchsvoller, da die bei der Trennung anfallenden Zusammensetzungen über die gesamte Destillationsvorrichtung ausreichend polymerisationsinhibiert werden müssen. Außerdem sind tert-Butylester im Vergleich zu niederen Estern relativ hochsiedend, was die thermische Belastung bei der Destillation erhöht.

Die DE 10 2008 002 923 A1 beschreibt ein Verfahren zur Herstellung tertiärer Alkylester (Meth)acrylsäure aus (Meth)acrylsäure und einem Olefin, wobei die (destillative) Abtrennung des Esters von der (Meth)acrylsäure unter Stabilisierung mittels einer N-Oxylverbindung erfolgt.

Die DE 195 392 95 A1 beschreibt ein Verfahren zur kontinuierlichen destillativen Auftrennung von flüssigen Gemischen, die als Hauptbestandteil (Meth)acrylsäure enthalten, in einer Destillationsvorrichtung. Ein Teilstrom der in die Destillationsvorrichtung geleiteten Flüssigkeit wird entnommen und in überhitzter Form in die Destillationsvorrichtung zurückgeführt. Die aufgereinigte (Meth)acrylsäure wird der Destillationsvorrichtung über Kopf entnommen.

Copolymere von tert-Butyl(meth)acrylat finden unter anderem Anwendung zur Herstellung von vernetzend härtenden Lackformulierungen, die beispielsweise als Fahrzeuglacke oder Reparaturlacke dienen. Hierfür ist ein niedriger Säuregehalt erwünscht.

Die Aufgabe wird gelöst durch ein Verfahren zur kontinuierlichen Herstellung des tert-Butylesters einer ethylenisch ungesättigten Carbonsäure, bei dem man
a) eine ethylenisch ungesättigte Carbonsäure mit Isobuten in Gegenwart eines sauren Katalysators zu einem Veresterungsgemisch umsetzt;
b) den sauren Katalysator abtrennt;
c) leichtsiedende Komponenten abtrennt; und
d) eine tert-Butylester enthaltende Flüssigkeit einer Destillationsvorrichtung zuführt und in der Destillationsvorrichtung reindestilliert, wobei man
d1) die tert-Butylester enthaltende Flüssigkeit in der Destillationsvorrichtung in ein tert-Butylester enthaltendes gasförmiges Kopfprodukt und ein Carbonsäure enthaltendes flüssiges Sumpfprodukt auftrennt;
d2) das tert-Butylester enthaltende gasförmige Kopfprodukt zumindest teilweise kondensiert und das Kondensat teilweise als Rücklauf in die Destillationsvorrichtung zurückführt;
d3) das Carbonsäure enthaltende flüssige Sumpfprodukt zumindest teilweise in Schritt a) zurückführt;
d4) Carbonsäure enthaltendes flüssiges Sumpfprodukt abzieht und zu einem Aufheizer leitet, einen überhitzten, flüssigen Rückführstrom aus dem Aufheizer entnimmt und den überhitzten Rückführstrom in die Destillationsvorrichtung entspannt; und
d5) zumindest im Kopfbereich der Destillationsvorrichtung die mit dem Brüden in Kontakt stehenden Wände der Destillationsvorrichtung zumindest in Teilbereichen beheizt und/oder thermisch isoliert.

Als Sumpfprodukt wird ein im unteren Teil der Destillationsvorrichtung entnommenes, flüssiges Produkt verstanden, z. B. eine am geodätisch tiefsten Punkt der Destillationsvorrichtung oder den unmittelbar darüber liegenden ein bis drei Böden entnommene Flüssigphase. Als Kopfprodukt wird ein im oberen Teil der Destillationsvorrichtung entnommenes, gasförmiges oder flüssiges Produkt verstanden, z. B. eine am Kopf der Destillationsvorrichtung oder den unmittelbar darunter liegenden ein bis drei Böden entnommene Flüssigphase.

Bei der erfindungsgemäß durchgeführten Erhitzung und Rückführung des abgezogenen flüssigen Sumpfproduktes wird die Bildung eines freien Gas-Dampfraums im gesamten Leitungsweg zum und vom Aufheizer vermieden. So werden Gasphasen, welche im Leitungsweg kondensieren und nicht-stabilisierte, polymerisationsanfällige Flüssigphasen bilden können, im Wesentlichen vermieden.

Bei dem erfindungsgemäßen Verfahren werden außerdem die Wände, die mit dem Brüden in Kontakt stehen, zumindest im Kopfbereich der Destillationsvorrichtung zumindest in Teilbereichen beheizt und/oder thermisch isoliert. Durch diese Maßnahme wird die Temperatur der Wände in diesem Bereich vorzugsweise über der Kondensationstemperatur des tert-Butylesters gehalten. So wird eine Kondensation des Brüdens an den Wänden vermieden, welche die Bildung nicht-stabilisierter polymerisationsanfälliger Flüssigphasen bedingen kann.

Als Kopfbereich der Destillationsvorrichtung wird der von Einbauten freie Bereich oberhalb des obersten Bodens bzw. oberhalb der obersten Packungsschicht bezeichnet. Er wird in der Regel von einem gewölbten Boden (Haube, z. B. Klöpperboden oder Korbbogenboden) gebildet, welcher das Abschlusselement der Destillationsvorrichtung bildet.

Als Kondensationstemperatur einer Verbindung wird die Temperatur bezeichnet, ab der die Verbindung bei einem gegebenen Druck kondensiert, das heißt vom gasförmigen in den flüssigen Aggregatzustand übergeht.

Bei der Veresterung wird eine ethylenisch ungesättigte Carbonsäure mit Isobuten in Gegenwart eines sauren Katalysators zu einem Veresterungsgemisch umgesetzt. Die ethylenisch ungesättigte Carbonsäure ist bevorzugt ausgewählt unter Acrylsäure, Methacrylsäure, Dimethylacrylsäure, Ethylacrylsäure, Allylessigsäure, Vinylessigsäure und Vinylpropionsäure. In einer bevorzugten Ausführungsform handelt es sich um Acrylsäure oder Methacrylsäure, wobei Methacrylsäure besonders bevorzugt ist.

In einer bevorzugten Ausführungsform weist die ethylenisch ungesättigte Carbonsäure einen Essigsäuregehalt von weniger als 300 ppm, besonders bevorzugt weniger als 100 ppm, ganz besonders bevorzugt weniger als 20 ppm und einen Propionsäuregehalt von weniger als 300 ppm, besonders bevorzugt weniger als 200 ppm, ganz besonders bevorzugt weniger als 130 ppm auf. Da Essigsäure und Propionsäure im weiteren Verfahren nur unter hohem Aufwand vom tert-Butylester abzutrennen sind, ist es bevorzugt, ethylenisch ungesättigte Carbonsäure mit kontrolliertem Gehalt an Essig- bzw. Propionsäure einzusetzen, falls ein ethylenisch ungesättigter tert-Butylester mit spezifiziertem Gehalt an Essigsäure bzw. Propionsäure angestrebt wird. Der Gehalt an Essigsäure und Propionsäure wird üblicherweise mittels Gaschromatographie bestimmt.

Die Veresterung erfolgt im Allgemeinen in Abwesenheit eines Lösungsmittels und in flüssiger Phase. Als Katalysatoren verwendet man daher solche, die im Reaktionsgemisch zumindest teilweise löslich sind. Geeignete Katalysatoren sind starke anorganische oder organische Säuren. Starke anorganische Säure sind beispielsweise Mineralsäuren wie Schwefelsäure, Phosphorsäure und Polyphosphorsäure, vorzugsweise Schwefelsäure. Starke organische Säuren sind beispielsweise Sulfonsäuren, wie p-Toluol-, Benzol-, Dodecylbenzol- und Methansulfonsäure, vorzugsweise p-Toluolsulfonsäure und Methansulfonsäure. Insbesondere die anorganischen Katalysatoren sind zu Beginn der Umsetzung nur teilweise im Reaktionsgemisch löslich. Im Laufe der Umsetzung wird der Katalysator besser löslich (in erster Linie auf Grund der Bildung eines Teilesters des Katalysators, z. B. des Schwefelsäurehalbesters). Zumindest im letzten Abschnitt liegt er daher im Allgemeinen im Reaktionsgemisch gelöst vor.

Die Konzentration des Katalysators im Veresterungsgemisch beträgt im Allgemeinen etwa 0,1 bis 10Gew.-%, vorzugsweise 0,5 bis 5Gew.-%, bezogen auf die Gesamtmenge des Veresterungsgemischs.

Die Umsetzung der ethylenisch ungesättigten Carbonsäure mit Isobuten in Gegenwart eines sauren Katalysators erfolgt bevorzugt in herkömmlichen Reaktionsbehältern oder in Säulen (DE-A-11 28 428). Ein geeigneter Reaktor ist in der WO 02/10109 A1 beispielhaft beschrieben.

Bevorzugt wird die Umsetzung in einem Reaktor durchgeführt, bei dem es sich insbesondere um einen zylindrischen Reaktor handelt. Der Reaktor ist in mehrere, vorzugsweise 3, 4 oder 5, voneinander getrennte Abschnitte unterteilt. Die Abschnitte werden durch Trennwände, die senkrecht zur Längsachse des Reaktors verlaufen, voneinander getrennt. Diese weisen jeweils mindestens eine Öffnung auf, um den Durchtritt des Reaktionsgemisches von einem Reaktorabschnitt zum nächsten zu ermöglichen. Die Zahl der Öffnungen pro Trennwand richtet sich nach der Größe des Reaktors. Vorzugsweise weisen die Trennwände eine Öffnung auf, die sich insbesondere in der Mitte der Trennwand befindet. Die Gesamtfläche der Öffnungen pro Trennwand beträgt etwa 1/2000 bis 1/500 der Reaktorquerschnittsfläche.

Das Volumen der Reaktorabschnitte kann gleich oder verschieden sein. Vorzugsweise ist das Volumen des ersten Reaktorabschnitts größer als das der restlichen Abschnitte. Bei einem Reaktor mit vier Abschnitten haben sich folgende Anteile der einzelnen Abschnitte am Gesamtreaktorvolumen als bevorzugt erwiesen:

| | |
|---|---|
| Reaktorabschnitt 1: | 25 bis 50% |
| Reaktorabschnitt 2: | 10 bis 25% |
| Reaktorabschnitt 3: | 10 bis 25% |
| Reaktorabschnitt 4: | 25 bis 50% |

Die Reaktorabschnitte können vorteilhaft mit Einbauten ausgestattet sein, um die Durchmischung des Reaktionsvolumens zu verbessern. Geeignete Einbauten sind beispielsweise statische Mischelemente und ähnlich wirkende Einbauten, wie Gitterroste, Verteilerbleche oder Siebböden. Besonders bevorzugt ist es, den ersten Reaktorabschnitt mit derartigen Einbauten auszurüsten, die dort dann insbesondere in der oberen Hälfte des Reaktorabschnittes vorgesehen werden.

Die Carbonsäure wird in flüssiger Form in den ersten Abschnitt des Reaktors eingespeist, insbesondere im Bereich des Bodens des Reaktors. Die Einspeisung kann direkt, z. B. über ein Tauchrohr erfolgen, es ist jedoch bevorzugt, Mittel vorzusehen, welche eine gleichmäßige Verteilung und Durchmischung der Einsatzstoffe ermöglichen. Derartige Mittel sind dem Fachmann bekannt, beispielsweise handelt es sich um Verteilerplatten, perforierte Platten und Rohre, Düsen, etc. Die Carbonsäure wird vorzugsweise über eine Düse eingespeist, welche die Vermischung eines Gases und einer Flüssigkeit und die Durchmischung des Reaktorinhalts bewirkt. Sie ist vorzugsweise im Boden des Reaktors angeordnet. Geeignete Düsen sind dem Fachmann bekannt (Strahldüse, Mischdüse, Zweistoffdüse, etc.) und z. B. in Ullmann's Encyclopedia of Industrial Chemistry, Vol. B4, 5. Ausgabe, 1992, S. 280 beschrieben. Insbesondere bei Verwendung einer derartigen Düse ist die Strömung in den beiden ersten Reaktorabschnitten turbulent und in den folgenden Reaktorabschnitten aber im Wesentlichen laminar. Dies erlaubt die Kaskadierung von Reaktionsabschnitten unterschiedlicher Charakteristik, z. B. turbulent mit hoher Rückvermischung wie beim Typ Rührkessel oder laminar mit geringer Rückvermischung wie beim Typ Rohrreaktor, wodurch der jeweilige Reaktionsabschnitt besonders vorteilhaft gestaltet werden kann.

Der Katalysator wird im Gemisch mit der Carbonsäure eingespeist, wobei frischer Katalysator oder zurückgewonnener Katalysator oder ein Gemisch davon zur Anwendung kommen kann.

Es hat sich als vorteilhaft erwiesen, zumindest einen Teil der flüssigen Schwersiederphase aus der nachstehend beschriebenen Katalysatorabtrennung und/oder zumindest einen Teil des Sumpfprodukts der Reindestillation in den Reaktor einzuspeisen. Auf diese Weise werden eine Hauptmenge des sauren Katalysators und der nicht umgesetzten Carbonsäure recycliert.

Das Isobuten kann flüssig und/oder gasförmig eingespeist werden. Es wird vorzugsweise über ein ringförmiges Rohr mit mehreren Austrittsöffnungen eingespeist.

Aus dem ersten und/oder zweiten Reaktorabschnitt kann ein Teil des Reaktionsgemisches entnommen und wieder in den betreffenden Abschnitt zurückgeführt werden. Dadurch wird eine bessere Durchmischung des Reaktionsgemisches gewährleistet. Die Rückführung des Teilstroms erfolgt zweckmäßig über die oben erwähnte Mischdüse in den ersten Reaktorabschnitt und/oder über eine weitere Düse im Bereich der in der Trennwand befindlichen Öffnung in den zweiten Reaktorabschnitt. Bei der weiteren Düse kann es sich um eine Düse des oben für die Mischdüse genannten Typs handeln. Bevorzugt wird eine kegelförmige Düse verwendet. Vorzugsweise ist sie so angeordnet, dass sich ihre Austrittsöffnung etwa in Höhe der Trennwand befindet, die den ersten vom zweiten Abschnitt trennt. Zur Kontrolle bzw. Regelung der Temperatur kann der jeweils entnommene Teilstrom über einen Wärmeübertrager geführt werden.

Das erhaltene Veresterungsgemisch wird am oberen Ende des Reaktors entnommen und der weiteren Aufarbeitung zugeführt. Nicht umgesetztes, gasförmiges Isobuten sammelt sich im oberen Bereich des Reaktors an. Vorzugsweise werden aus dem am oberen Ende des Reaktors abgezogenen Isobuten-haltigen Gasstrom kondensierbare organische Verbindungen, wie nicht umgesetzte Carbonsäure, auskondensiert und so von gegenüber der Veresterung inerten Gasen, wie Luft und Butan, befreit. Nicht umgesetztes Isobuten löst sich teilweise in den auskondensierten Bestandteilen. Die kondensierten organischen Verbindungen werden dann in flüssiger Form beispielsweise über die Mischdüse in den ersten Reaktorabschnitt eingespeist.

Die Veresterungstemperatur liegt insgesamt im Bereich von etwa 10 bis 40 °C. Sie wird vorzugsweise so gesteuert, dass sie im ersten Reaktorabschnitt am höchsten ist. Vorzugsweise liegt die Reaktionstemperatur im ersten Reaktorabschnitt im Bereich von etwa 30 bis 40 °C. Im zweiten Abschnitt ist sie niedriger, vorzugsweise um etwa 5 bis 15 °C. Die Temperatur in den nach dem zweiten Abschnitt folgenden Abschnitten kann gleich oder verschieden sein. Sie ist im Allgemeinen nicht höher als im zweiten Abschnitt, vorzugsweise ist sie niedriger, insbesondere um etwa 3 bis 10 °C. Im vierten Abschnitt ist sie im Allgemeinen so hoch wie im dritten Abschnitt oder um etwa 1 bis 5 °C niedriger. Die Temperatur im letzten Reaktorabschnitt liegt vorzugsweise im Bereich von etwa 10 bis 25 °C.

Die Temperaturverteilung in einem Reaktor mit 4 Abschnitten ist vorzugsweise wie folgt:

| | |
|---|---|
| 1. Abschnitt: | 33 bis 38 °C |
| 2. Abschnitt: | 23 bis 28 °C |
| 3. Abschnitt: | 15 bis 22 °C |
| 4. Abschnitt: | 15 bis 22 °C |

Die Temperatur im 3. und 4. Abschnitt kann dabei gleich oder verschieden sein.

Da die Addition von Carbonsäuren an Isobuten exotherm ist, ist es zur Einstellung der Reaktionstemperatur zweckmäßig, zumindest in den ersten beiden Reaktorabschnitten die Reaktionswärme abzuführen. Dies erfolgt insbesondere mit Hilfe von Wärmetauschern, die außen- oder innenliegend ausgeführt sein können. Auch eine Kühlung der Reaktorwände ist möglich. Es hat sich als zweckmäßig erwiesen, die Temperaturkontrolle in den beiden ersten Reaktorabschnitten mit Hilfe von außenliegenden Wärmetauschern vorzunehmen, über die ein Teilstrom des in dem jeweiligen Reaktorabschnitt befindlichen Reaktionsgemisches geführt und wieder zurückgeführt wird.

Die Veresterung kann mit Unterdruck, drucklos oder unter leichtem Überdruck (100 bis 300 mbar abs.), oder vorzugsweise mit Überdruck (z. B. 0,5 bis 3 bar) durchgeführt werden.

Das aus dem Reaktor austretende Reaktionsgemisch enthält einen hohen Anteil an dem gewünschten Ester. Daneben enthält es nicht umgesetzte Reaktanden, Katalysator, Stabilisator, Ester der Katalysatorsäure und weitere geringfügige Nebenprodukte. Das Reaktionsgemisch enthält nur sehr geringe Mengen an Isobuten-Oligomerisierungsprodukt, im Allgemeinen < 2 Gew.-%, bezogen auf das Reaktionsgemisch.

Aus dem Veresterungsgemisch wird zunächst der saure Katalysator abgetrennt. Zur Abtrennung des sauren Katalysators wird das Veresterungsgemisch bevorzugt partiell verdampft, wodurch man eine den sauren Katalysator enthaltende flüssige Schwersiederphase und einen tert-Butylester und Isobuten enthaltenden Brüden erhält. Die flüssige Schwersiederphase wird im Allgemeinen zumindest teilweise in den Reaktor zurückgeführt.

Die partielle Verdampfung kann auf beliebige Weise durchgeführt werden, wird aber bevorzugt zweistufig durchgeführt. Die Verdampfung erfolgt im Allgemeinen bei erhöhter Temperatur und bei verringertem Druck. Die Bedingungen richten sich nach dem jeweils gewünschten Produkt. Sie werden in der Regel so gewählt, dass die Temperatur im Bereich von etwa 50 bis 150 °C liegt. Der Druck wird so eingestellt, dass die Verdampfung rasch und schonend erfolgt. Der Druck liegt z.B. im Bereich von 10 bis 200 mbar abs., besonders bevorzugt im Bereich von 30 bis 90 mbar abs., ganz besonders bevorzugt im Bereich von 50 bis 70 mbar abs.

Zur Erzeugung des Vakuums sind jegliche Vakuumpumpen geeignet. Zur Vermeidung von Kontaminationen hat es sich bewährt, Schmieröl-freie Pumpen zu verwenden. Besonders bevorzugt werden Wälzkolbenvakuumpumpen ohne Schmieröl und sogenannte trockenlaufende Schrauben-Vakuumpumpen verwendet. Alternativ können Flüssigkeitsringpumpen verwendet werden, in denen z. B. der Zielester als Sperrflüssigkeit dient.

Die zweistufige Verdampfung wird vorzugsweise so durchgeführt, dass in einer ersten Stufe 40 bis 95 Gew.-%, vorzugsweise 60 bis 90 Gew.-% des gewünschten Esters abdampfen. Der Brüden enthält neben dem tert-Butylester und Carbonsäure die leichtsiedenden Bestandteile, wie tert-Butanol, tert-Butylacetat und Diisobuten. Der in der ersten Destillation anfallende Sumpf umfasst als erste Schwersiederphase im Wesentlichen den restlichen tert-Butylester, Carbonsäure, sauren Katalysator und hochsiedende Bestandteile, beispielsweise polymere (Meth)acrylverbindungen bei Verwendung von (Meth)acrylsäure. 10 bis 100 Gew.-% der ersten Schwersiederphase werden der zweiten Verdampfungsstufe zugeführt. Falls nur ein Teil der ersten Schwersiederphase der zweiten Verdampfungsstufe zugeführt wird, wird der Rest der ersten Schwersiederphase in den Reaktor zurückgeführt. In der zweiten Verdampfungsstufe werden der restliche Zielester und die Hauptmenge an Carbonsäure (bis zu etwa 90 Gew.-%) abgedampft.

Der Sumpf der zweiten Verdampfungsstufe umfasst als zweite Schwersiederphase im Wesentlichen den sauren Katalysator, die restliche Carbonsäure und hochsiedende Bestandteile. Die zweite Schwersiederphase wird zumindest teilweise ausgeschleust, vorzugsweise vollständig. Sie kann jedoch auch teilweise in den Reaktor zurückgeführt werden. Die Brüden beider Stufen werden vereinigt und kondensiert. Das Destillat enthält im Allgemeinen < 20 ppm, insbesondere < 10 ppm, Katalysator.

Beide Verdampfungsstufen können in üblichen Vorrichtungen durchgeführt werden. Vorzugsweise verwendet man jedoch Vorrichtungen, die eine rasche Destillation erlauben, beispielsweise Filmverdampfer. Geeignete Filmverdampfer sind dem Fachmann bekannt, siehe beispielsweise Ullmann's Encyclopedia of Industrial Chemistry, 5th Ed., Vol. B3,2-21 bis 2-24 sowie 3-1 bis 3-25,1988.

Vorzugsweise verwendet man in der ersten Verdampfungsstufe Fallfilm- oder Fallstromverdampfer und in der zweiten Stufe Dünnschichtverdampfer mit Wischern oder Wendelrohrverdampfer.

Als besonders bevorzugt hat es sich erwiesen, in der ersten Verdampfungsstufe einen wie in der WO 02/10110 beispielhaft beschriebenen Fallfilmverdampfer zu verwenden.

Der Brüden, welcher im Wesentlichen den Zielester, Carbonsäure und leichtsiedende Bestandteile, wie tert-Butanol, tert-Butylacetat und Diisobuten enthält, wird üblicherweise kondensiert. Die Kondensation erfolgt vorzugsweise in einem oder mehreren in Serie geschalteten Platten- oder Rohrbündelkondensatoren. Vorzugsweise verwendet man Rohrbündelkondensatoren mit senkrecht angeordneten Rohren, die vom Brüden von oben nach unten angeströmt werden. Nicht-kondensierter Brüden wird bevorzugt in den Reaktor zurückgeführt.

In einer Ausführungsform erfolgt die Kondensation des Brüdens als zweistufige Kondensation, bei der eine Hauptmenge des Esters bei einer ersten Temperatur kondensiert wird und eine weitere Menge des Esters bei einer zweiten Temperatur kondensiert wird, die niedriger ist als die erste Temperatur. Hierdurch wird eine möglichst vollständige Abtrennung des tert-Butylesters bei möglichst geringer Co-Kondensation von nicht umgesetztem Isobuten erreicht. Die fraktionierende Kondensation erfolgt bevorzugt in zwei in Serie geschaltete Kondensatoren.

Zur Vermeidung von Feststoffablagerungen an den Kontaktflächen des Kondensators wird zweckmäßigerweise ein Teilstrom des gesammelten Kondensats umgewälzt, um die Kontaktflächen fortwährend zu spülen. Das umgepumpte Kondensat kann z. B. mittels eines Verteilers gleichmäßig über die Rohre des Rohrbündelkondensators verteilt werden und läuft an den Innenwänden der Rohre des Kondensators hinab. Beim Einsatz polymerisationsfähiger Carbonsäuren wird auf diese Weise auch eine Verteilung eines nachstehend näher beschriebenen Stabilisators erzielt.

Das (vereinigte) Kondensat der Kondensation des Brüdens wird einer Leichtsiederabtrennung zugeführt, welche bevorzugt destillativ durchgeführt wird. Bei der destillativen Leichtsiederabtrennung werden die leichtsiedenden Bestandteile, d. h. von Isobuten verschiedene Bestandteile, deren Siedepunkt niedriger ist als der des Zielesters, vorzugsweise über Kopf abdestilliert. Bei der Leichtsiederabtrennung fallen außerdem weitere Mengen nicht umgesetztes Isobuten an, die vorzugsweise von den leichtsiedenden Bestandteilen abgetrennt werden und in den Schritt zurückgeführt werden. Bei der Herstellung von tert-Butylmethacrylat fallen als Leichtsieder beispielsweise tert-Butylacetat, tert-Butanol und Diisobuten an.

Zur Leichtsiederabtrennung führt man Kondensat aus der Katalysator-Abtrennung einer Leichtsieder-Destillationskolonne zu, wobei man im Sumpf ein von Leichtsiedern befreites flüssiges Produkt und am Kopf einen Leichtsieder-Brüden erhält. Das von Leichtsiedern befreite flüssige Produkt, welches im Wesentlichen Zielester und Carbonsäure umfasst, wird einer weiteren Aufarbeitung zugeführt. Der Leichtsieder-Brüden wird kondensiert. Bevorzugt wird das Kondensat teilweise als Rücklauf zum Kopf der Leichtsieder-Destillationskolonne zurückgeführt.

Nicht-kondensierter Leichtsieder-Brüden kann noch bis zu 5 Gew.-%, bezogen auf das Kopfprodukt, an Zielester enthalten. Der Leichtsieder-Brüden enthält außerdem Isobuten und wird bevorzugt der Veresterung zugeführt.

Die Destillationstemperatur (Sumpftemperatur) in der Leichtsieder-Destillationskolonne liegt im Allgemeinen im Bereich von 30 bis 110 °C. Der Druck wird entsprechend je nach Produkt gewählt. Bei der Herstellung von tert-Butylmethacrylsäureester liegt der Druck beispielsweise im Bereich von 0,1 bis 0,25 bar (abs).

Als Leichtsieder-Destillationskolonne kommen übliche Kolonnen mit Schüttungen oder gerichteten Packungen oder mit Glocken-, Ventil- oder Siebböden in Betracht. Vorzugsweise verwendet man jedoch eine Bodenkolonne mit 30 bis 50 Dual-Flow-Böden. Der Zulauf zur Leichtsieder-Destillationskolonne erfolgt im Allgemeinen im mittleren Bereich.

Die Kondensation der leichtsiedenden Komponenten erfolgt vorzugsweise in einem oder mehreren in Serie geschalteten Kondensatoren, insbesondere Platten- oder Rohrbündelkondensatoren. Vorzugsweise verwendet man Rohrbündelkondensatoren mit senkrecht angeordneten Rohren, die vom Brüden von oben nach unten angeströmt werden.

Diisobuten ist Hauptbestandteil der abgetrennten Leichtsieder. Diisobuten ist ein Gemisch verschiedener Isoocten-Isomerer. Deren Kondensationspunkt liegt in der Praxis eng beisammen. Als Bezugspunkt kann z. B. der Kondensationspunkt von 2,4,4-Trimethylpent-1-en gelten.

In einer Ausführungsform erfolgt die Kondensation des Leichtsieder-Brüdens als zweistufige Kondensation, bei der eine Hauptmenge des Diisobutens bei einer ersten Temperatur kondensiert wird und eine weitere Menge des Diisobutens bei einer zweiten Temperatur kondensiert wird, die niedriger ist als die erste Temperatur. Hierdurch wird eine möglichst vollständige Abtrennung des Diisobutens bei möglichst geringer Co-Kondensation von nicht umgesetztem Isobuten erreicht. Die fraktionierende Kondensation erfolgt bevorzugt in zwei in Serie geschaltete Kondensatoren.

Nach der Abtrennung des sauren Katalysators (Stufe b) sowie der leichtsiedenden Komponenten (Stufe c) aus dem Veresterungsgemisch wird die tert-Butylester enthaltende Flüssigkeit in einer Destillationsvorrichtung reindestilliert (Stufe d).

Bei der Reindestillation wird die tert-Butylester enthaltende Flüssigkeit in der Destillationsvorrichtung in ein tert-Butylester enthaltendes gasförmiges Kopfprodukt und ein Carbonsäure enthaltendes flüssiges Sumpfprodukt aufgetrennt. Das Sumpfprodukt wird zumindest teilweise, insbesondere vollständig in die Veresterung zurückgeführt.

Die Destillationstemperatur liegt im Allgemeinen im Bereich von 40 bis 130 °C. Der Druck wird entsprechend dem zu destillierenden Ester gewählt.

Bei der Destillationsvorrichtung handelt es sich üblicherweise um eine Destillationskolonne, beispielsweise um eine herkömmliche Bodenkolonne, z. B. eine Kolonne mit 30 bis 50 Dual Flow-Böden und Zulauf im mittleren Kolonnenbereich. Der im Wesentlichen reine Zielester wird über den Kopf der Kolonne abgetrennt. Das erfindungsgemäße Verfahren kann als kontinuierliche Destillation, z. B. in einer Destillationskolonne, aber auch als absatzweise durchgeführte Destillation, z. B. in einer Destillationsblase, durchgeführt werden.

Bei der Reindestillation werden zumindest die Wände im Kopfbereich der Destillationsvorrichtung beheizt und/oder thermisch isoliert. Durch das Beheizen bzw. durch die thermische Isolierung der Wände im Kopfbereich wird eine Kondensation gasförmiger Produkte an den Wänden vermieden, welche die Bildung nicht-stabilisierter polymerisationsanfälliger Flüssigphasen bedingen kann.

In der Regel ist die Destillationsvorrichtung so ausgestaltet, dass eine Haube mit dem zylindrischen Teil der Destillationsvorrichtung über einen Flansch verbunden ist. Der Flansch stellt eine Kältebrücke dar; so können niedrigere Temperaturen als im Rest des Kopfbereichs herrschen, wodurch ein erhöhtes Kondensationspotential herrscht. Eine zusätzliche Polymerisationsinhibierung ist daher an dieser Stelle vorteilhaft.

Bevorzugt wird in das Brüdenrohr, über welches das tert-Butylester enthaltende gasförmige Kopfprodukt aus der Destillationsvorrichtung ausgeführt wird, ein Polymerisationsinhibitor dosiert. Vorzugsweise erfolgt die Dosierung über ein Durchflussbegrenzungsmittel, vorzugsweise eines, das eine Flüssigkeitsfeinverteilung gestattet. Bevorzugt wird als Durchflussbegrenzungsmittel eine Blende, ein Ventil, eine Drossel, eine Lochscheibe, eine Düse, eine Kapillare oder Kombinationen davon, insbesondere eine Düse, verwendet. Die Verwendung eines Durchflussbegrenzungsmittels erlaubt eine verbesserte Verteilung des Stabilisators an den Wänden im Kopfbereich der Destillationsvorrichtung sowie am Flansch, der die Haube und den zylindrischen Teil der Destillationsvorrichtung verbindet.

Aufgrund der unterschiedlichen Flüchtigkeiten der zu trennenden Substanzen stellen sich über die Länge der Kolonne Konzentrationsprofile ein. So dominiert im oberen Bereich der Destillationsvorrichtung der tert-Butylester und im unteren Bereich die nicht umgesetzte ethylenisch ungesättigte Carbonsäure. Auch zur Stabilisierung eingesetzte Polymerisationsinhibitoren weisen unterschiedliche Flüchtigkeiten auf. Der Siedepunkt der Polymerisationsinhibitoren ist in der Regel höher als der des tert-Butylesters. So kann es dazu kommen, dass tert-Butylester bzw. Carbonsäure verdampfen und an einer kühleren Stelle der Destillationsvorrichtung kondensieren. Die Kondensate sind hier potentiell unzureichend polymerisationsinhibiert. Der Bildung dieser Kondensate wird durch ein Beheizen der Wände im Kopfbereich der Destillationsvorrichtung und/oder deren thermischer Isolierung vorgebeugt. Die Feinverteilung des Polymerisationsinhibitors im Brüdenrohr führt außerdem zu einer gleichmäßigeren Verteilung des Polymerisationsinhibitors an den Wänden im Kopfbereich der Destillationsvorrichtung und somit zu weniger polymerisationsanfälligen Kondensaten.

Die Beheizung der Wände im Kopfbereich der Destillationsvorrichtung erfolgt bevorzugt dadurch, dass man den zu beheizenden Bereich mit einer Ummantelung thermisch in Kontakt bringt, welche mit einem Heizmedium, beispielsweise Heißdampf, durchströmt wird.

Vorzugsweise handelt es sich bei der Ummantelung um einen Doppelmantel, eine Halbrohrschlange, oder Begleitheizungsschläuche oder -leitungen, insbesondere eine Halbrohrschlange. Unter einer Halbrohrschlange wird ein halbschalen-förmiges Rohrsegment verstanden, welches um den zu beheizenden Bereich gelegt und mit dessen Außenwand verschweißt wird. Die Halbrohrschlange wird bevorzugt mit Heißdampf bei einem Druck von 1,2 bis 2,5 bar, besonders bevorzugt 1,3 bis 1,7 bar, beispielsweise 1,5 bar betrieben. Üblicherweise ist der Druck regulierbar. Die Temperatur liegt in der Regel im Bereich von 105 bis 130 °C, bevorzugt im Bereich von 108 bis 113 °C.

Alternativ kann auch eine elektrische Beheizung, z. B. mittels Heizdrähten oder Heizmatten, verwendet werden.

Die Temperatur der Wände im Kopfbereich der Destillationsvorrichtung kann auch mittels thermischer Isolierung oberhalb der Kondensationstemperatur des tert-Butylesters gehalten werden. Eine aktive Beheizung der Wände im Kopfbereich der Destillationsvorrichtung ist jedoch bevorzugt.

Die thermische Isolierung der Wände im Kopfbereich der Destillationsvorrichtung kann mittels Aufbringung üblicher Isolationsmaterialien auf die Außenwand des Kopfbereichs, wie (Glas)wolle, Verbundwerkstoffe, Tafeln oder Platten, oder Rohren erfolgen.

Die Temperatur der beheizten Wände liegt bevorzugt im Bereich von 2 bis 20 °C, besonders bevorzugt 2 bis 15 °C, beispielsweise 5 bis 10 °C oberhalb der Kondensationstemperatur des tert-Butylesters bei dem in der Destillationsvorrichtung herrschenden Druck.

Das tert-Butylester enthaltende gasförmige Kopfprodukt wird zumindest teilweise kondensiert. Das Kondensat wird teilweise als Rücklauf in die Destillationsvorrichtung zurückgeführt. Der andere Teil des Kondensats wird als Produkt aus dem Verfahren ausgeführt.

Je höher das Rücklauf-Verhältnis in der Destillationsvorrichtung, desto besser fällt die Trennung von ethylenisch ungesättigter Carbonsäure und dem tert-Butylester aus. Unter dem Rücklauf-Verhältnis wird das Verhältnis der Menge an in die Destillationsvorrichtung zurückgeführtem Kondensat des tert-Butylester enthaltenden gasförmigen Kopfproduktes zur Menge an weitergeführtem Kondensat des tert-Butylester enthaltenden gasförmigen Kopfproduktes verstanden. Unter Verwendung eines hohen Rücklauf-Verhältnisses kann somit ein Carbonsäure-armer tert-Butylester erhalten werden. Ein hohes Rücklauf-Verhältnis bedingt allerdings eine hohe Sumpftemperatur und eine höhere thermische Belastung der ethylenisch ungesättigten Carbonsäure und des tert-Butylesters. Das Rücklauf-Verhältnis liegt bevorzugt im Bereich von 2 bis 4, besonders bevorzugt im Bereich von 2,5 bis 3,5. Insbesondere unter diesen Bedingungen kommen die Vorteile des erfindungsgemäßen Verfahrens zum Tragen.

Das Endprodukt weist vorzugsweise einen Gesamtgehalt an Essigsäure, Propionsäure und ethylenisch ungesättigter Carbonsäure von weniger als 300 ppm, besonders bevorzugt weniger als 200 ppm, ganz besonders bevorzugt weniger als 130 ppm auf. Insbesondere bevorzugt weist das Endprodukt einen Gesamtgehalt an Säure von weniger als 300 ppm, besonders bevorzugt weniger als 200 ppm, ganz besonders bevorzugt weniger als 130 ppm auf. Für die Bestimmung des Säuregehalts wird die Säurezahl üblicherweise titrimetrisch bestimmt und als Gehalt an Methacrylsäure angegeben.

Die Kondensation des gasförmigen Kopfprodukts erfolgt vorzugsweise in einem oder mehreren in Serie geschalteten Kondensatoren, insbesondere Platten- oder Rohrbündelkondensatoren. Vorzugsweise verwendet man Rohrbündelkondensatoren mit senkrecht angeordneten Rohren, die vom Brüden von oben nach unten angeströmt werden.

Wird ein einzelner Kondensator verwendet, so liegt der Druck im Kondensator bevorzugt im Bereich von 10 bis 200 mbar abs., besonders bevorzugt im Bereich von 30 bis 100 mbar abs., ganz besonders bevorzugt im Bereich von 50 bis 90 mbar abs.. Die Temperatur im Kondensator bevorzugt 45 bis 80 °C, besonders bevorzugt 50 bis 65 °C, unterhalb der Kondensationstemperatur des tert-Butylesters beim verwendeten Druck. Die Temperatur liegt bevorzugt im Bereich von -25 bis 0 °C, besonders bevorzugt im Bereich von -20 bis -5 °C.

Beispielsweise beträgt die Kondensationstemperatur von tert-Butylacrylat bei 60 mbar abs. 43 °C. In diesem Fall wird der Kondensator zweckmäßigerweise mit Solekühlung betrieben verwendet.

In einer Ausführungsform erfolgt die Kondensation des gasförmigen Kopfprodukts als zweistufige Partialkondensation. Hierdurch wird eine möglichst vollständige Abtrennung des tert-Butylesters bei möglichst geringer Co-Kondensation von nicht abgetrennten Leichtsiedern erreicht. Die zweistufige Partialkondensation erfolgt bevorzugt in zwei in Serie geschaltete Kondensatoren. Die hier angegebenen Temperaturen der partiellen Kondensationen beziehen sich auf die Temperatur des Kondensats bei Entnahme aus dem jeweiligen Kondensator.

Die Temperatur des Kühlmittels des zweiten Kondensators ist um etwa 30 bis 60 °C niedriger als die des ersten Kondensators, bei dem das Kühlmittel eine Temperatur im Bereich von etwa 10 bis 35 °C aufweist.

Die erste Temperatur liegt bevorzugt 0 bis 45 °C, vorzugsweise 5 bis 35 °C, unterhalb der Kondensationstemperatur des tert-Butylesters beim ersten Druck und die zweite Temperatur 45 bis 80 °C, vorzugsweise 50 bis 65 °C, ganz besonders bevorzugt 50 bis 55 °C unterhalb der Kondensationstemperatur des tert-Butylesters beim zweiten Druck.

Die zweite Temperatur liegt mindestens 5 °C unterhalb der ersten Temperatur. Vorzugsweise liegt die zweite Temperatur mindestens 10 °C, besonders bevorzugt mindestens 20 °C, ganz besonders bevorzugt mindestens 30 °C und am bevorzugtesten mindestens 40 °C unterhalb der ersten Temperatur.

Der erste Druck liegt bevorzugt im Bereich von 10 bis 200 mbar abs., besonders bevorzugt im Bereich von 30 bis 100 mbar abs., ganz besonders bevorzugt im Bereich von 50 bis 90 mbar abs. Der zweite Druck liegt bevorzugt im Bereich von 10 bis 200 mbar abs., besonders bevorzugt im Bereich von 30 bis 100 mbar abs., ganz besonders bevorzugt im Bereich von 50 bis 90 mbar abs.. Meist sind der erste und zweite Kondensator gasseitig verbunden. Der erste und zweite Druck sind dann gleich.

Beispielsweise beträgt die Kondensationstemperatur von tert-Butylacrylat bei 60 mbar abs. 43 °C. In diesem Fall kann für die Kühlung des ersten Kondensators zweckmäßigerweise Flusswasser oder gleichwertig temperiertes Kühlwasser verwendet werden, während der zweite Kondensator mit Solekühlung betrieben verwendet wird.

Das (vereinigte) Kondensat wird teilweise als Produkt aus dem Verfahren ausgeführt.

Das Carbonsäure enthaltende flüssige Sumpfprodukt wird zumindest teilweise in die Veresterung zurückgeführt. Vorteilhafterweise wird das Carbonsäure enthaltende flüssige Sumpfprodukt, welches in die Veresterung zurückgeführt wird, auf eine Temperatur im Bereich von 25 bis 40 °C abgekühlt, bevorzugt 25 bis 35 °C. Üblicherweise wird hierfür ein Warmwasserkühler verwendet, welcher bei einer Temperatur im Bereich von 25 bis 35 °C, beispielsweise 30 °C, betrieben wird.

Im Anschluss an die Kühlung wird der Teilstrom des Sumpfstromes, welcher in die Veresterung zurückgeführt wird, bevorzugt über eine Vorrichtung zur Abtrennung von festen Verunreinigungen geführt. Bevorzugt handelt es sich hierbei um einen Filter. Auf diese Weise können ungelöste, feste Verunreinigungen, wie beispielsweise Polymerisatpartikel, weitgehend entfernt werden. So können Verstopfungen, beispielsweise in den Leitungen, vermieden werden. Zweckmäßig ist es, den Filter mit einer zuschaltbaren Bypassleitung zu versehen, um diesen zur Reinigung oder zum Austausch ohne Unterbrechung des Verfahrens entnehmen zu können.

Ein weiterer Teilstrom des flüssigen Sumpfproduktes wird über einen Zwangsumlaufverdampfer geleitet, also abgezogen und zu einem Aufheizer geleitet, aus welchem man einen überhitzten, flüssigen Rückführstrom entnimmt und in die Destillationsvorrichtung entspannt.

Der Druck auf das aus der Destillationsvorrichtung abgezogene, flüssige Sumpfprodukt, das zur Destillationsvorrichtung zurückgeführt wird, wird durch Überhitzen erhöht. Der überhitzte Rückführstrom wird über ein Durchflussbegrenzungsmittel entspannt. Hierdurch erfolgt eine Überhitzung der Flüssigkeit über ihren Siedepunkt in Bezug auf den Druck im Inneren der Destillationsvorrichtung.

Beim Durchtritt der überhitzten Flüssigkeit durch das Durchflussbegrenzungsmittel und Wiedereintritt in die Destillationsvorrichtung erfolgt eine schlagartige Verdampfung der Flüssigkeit. Diese schlagartige Verdampfung, die unter erheblicher Volumenvergrößerung verläuft, führt zu einer Beschleunigung des in die Destillationsvorrichtung eintretenden Fluidstroms, wodurch die Gefahr einer Bildung von Ablagerungen beispielsweise von Polymeren an der Eintrittsstelle in die Destillationsvorrichtung verringert wird. Vorteilhaft ist es daher, wenn das Durchflussbegrenzungsmittel unmittelbar vor dem Wiedereintritt der überhitzten Flüssigkeit in die Destillationsvorrichtung, oder sogar im Inneren dieser, angeordnet wird.

In einer Ausführungsform wird die Destillationsvorrichtung dabei in an sich bekannter Weise unter Eindosieren von Polymerisationsinhibitoren (Stabilisatoren) an geeigneten Stellen, beispielsweise am Kopf der Destillationsvorrichtung, betrieben. In dieser Weise wird vermieden, dass Dämpfe der leicht polymerisierbaren Verbindung bzw. Kondensate dieser Dämpfe in Behältern und Rohrleitungen auftreten, die nicht mit Stabilisatorlösungen benetzt sind.

Vorteilhaft ist es, dass durch Betrieb des Verdampfers im Zwangsumlauf eine gegenüber Betrieb mit Naturumlauf erhöhte Strömungsgeschwindigkeit der Flüssigkeit in der Erhitzungsvorrichtung, z. B. im Rohrbündel des Wärmetauschers, erzielt wird. Dadurch wird ein kontinuierliches Reinigen der Wärmeaustauschflächen durch das strömende Produkt erreicht. Ein weiterer Vorteil der Erfindung ist der durch die erhöhte Strömungsgeschwindigkeit verbesserte Wärmeübergang zwischen Wärmetauscher und erhitzter Flüssigkeit, der wiederum zur Vermeidung lokaler Überhitzungen beiträgt.

Bei der Durchführung des erfindungsgemäßen Verfahrens wird als Fördervorrichtung eine Pumpe verwendet, um den Betrieb des Aufheizers im Zwangsumlauf zu ermöglichen. Die Pumpe wird dabei bevorzugt zwischen der Entnahmeleitung und dem Aufheizer angeordnet, so dass der Druck auf den in Strömungsrichtung nachfolgenden Aufheizer erhöht wird, um ein Verdampfen der Flüssigkeit stromabwärts nach diesem zu verhindern. Eine weitere Pumpe kann zum Abführen des flüssigen Sumpfproduktstroms eingesetzt werden.

Es wird bevorzugt, dem in die Fördervorrichtung geführten Strom ein Anti-Fouling-Mittel, beispielsweise ausgewählt unter den unter dem Namen KOMAD vertriebenen Succinimid-Derivaten, zuzusetzen, um diese vor Kontamination, Verstopfung oder Beschädigung zu schützen. Auf diese Weise können ungelöste, feste Verunreinigungen, wie beispielsweise Polymerisatpartikel, weitgehend entfernt werden, bevor sie in den Aufheizer gelangen und dort zu Verstopfungen führen können.

Als Aufheizer können übliche Wärmetauscher zum Einsatz kommen, bevorzugt wird ein in indirektem Wärmetausch gegen ein Heizmedium arbeitender Wärmetauscher, insbesondere ein Rohrbündel-Wärmetauscher verwendet. Dieser wird bevorzugt in waagerechter Aufstellung betrieben. Auch die Verwendung von Plattenwärmetauschern oder Spiralwärmetauschern anstelle von Rohrbündel-Wärmetauschern ist möglich, da hier bei Einhaltung von entsprechenden Strömungsgeschwindigkeiten sowie unterdrückter Verdampfung die gleichen Vorteile wie beim Rohrbündel-Wärmetauscher erzielt werden können.

Möglich ist auch eine Beheizung der aus der Destillationsvorrichtung abgezogenen Flüssigkeit mit elektrischer Energie.

Bevorzugt wird als Durchflussbegrenzungsmittel eine Blende, ein Ventil, eine Drossel, eine Lochscheibe, eine Düse, eine Kapillare oder Kombinationen davon, insbesondere ein Ventil, verwendet. Beispielsweise kann ein Drehkegelventil verwendet werden. Besonders bevorzugt ist es, wenn die Öffnungscharakteristik des Durchflussbegrenzungsmittels verstellbar ist. Auf diese Weise kann der Druck im Verdampfer auch bei geänderten Strömungsgeschwindigkeiten, wie sie beispielsweise bei An- und Abfahrvorgängen auftreten können, immer über dem Siededruck der Flüssigkeit, bezogen auf den Druck im Inneren der Destillationsvorrichtung, gehalten werden.

Im stationären Zustand liegen im Reaktor die Edukte als Lösung im Zielester vor, was eine Vergleichmäßigung der Reaktion und eine besonders vorteilhafte Wärmeabfuhr erlaubt. Zum Anfahren des Reaktors wird der Reaktor daher bevorzugt mit dem Zielester befüllt. Im Anschluss werden die Edukte und der Katalysator in den Reaktor eingeleitet und die Reaktion beginnt.

Beim Abfahren der Anlage wird der Reaktorinhalt bevorzugt in einen Auffangbehälter geleitet. Der Auffangbehälter ist am geodätisch tiefsten Punkt der Anlage angeordnet und über separate Leitungen mit dem Reaktor verbunden. Im Falle einer Leckage ist so ein schnelles Entleeren des Reaktors möglich. Üblicherweise sind hierzu keine Pumpsysteme notwendig. Der Auffangbehälter verfügt über einen Druckausgleich und ist mit einem sauerstoffhaltigen Gas mit einem Sauerstoffgehalt von 10 Vol.-% Sauerstoff oder weniger, vorzugsweise 5 Vol.-% Sauerstoff oder weniger, in Inertgas, vorzugsweise Stickstoff, befüllt. Die Kühlung des Auffangbehälters erfolgt mittels einer Pumpe und einem externen Wärmetauscher. Der Inhalt des Auffangbehälters kann dann unabhängig weiter aufgearbeitet werden.

Die Edukte, insbesondere die Carbonsäure, werden vorzugsweise in weitgehend wasserfreier Form eingesetzt. Die im Verfahren mit den Reaktionskomponenten in Kontakt stehenden Oberflächen bestehen bevorzugt aus Materialien, die bezüglich technischer Korrosionsbeständigkeit an die Korrosivität der eingesetzten Carbonsäure angepasst sind, wie z.B. Edelstahl der Qualität 1.4541, 1.4571 bzw. diesen im Korrosionsverhalten mindestens gleichwertigen Edelstähle. Aufgrund des sehr niedrigen Wassergehalts im Prozesssystem kommt es bei diesen Werkstoffen auch bei Einsatz von starken anorganischen Säuren als Katalysator zu keinem über das Maß der technischen relevanten Beständigkeit hinausgehenden Korrosionsangriff. In Produktionsanlagen für ethylenisch ungesättigte Ester besteht üblicherweise die Notwendigkeit zur Reinigung mit heißer Natronlauge, wodurch die verwendeten Werkstoffe eine Wechselbelastung zwischen organischer Säure und dem Reinigungsmedium Natronlauge erfahren. Der Einsatz von sogenannten Duplexstählen wie 1.4462 für eine verbesserte Langzeitbeständigkeit der apparativen Ausrüstung kann daher von Vorteil sein.

Insbesondere in den Bereichen, wo neben der beschriebenen korrosiven Belastung durch organische Säuren und einer starken anorganischen Säure als Katalysator zusätzlich noch eine hohe Temperatur und eine mechanische Belastung vorliegen, wie im Dünnschichtverdampfer zur Abtrennung des sauren Katalysators vom Hauptteil der organischen Substanz, ist es von Vorteil, deutlich besser korrosionsbeständige Werkstoffe, z. B. Nickel-Basiswerkstoffe wie 2.4602, 2.4605, 2.4610 oder 2.4819, einzusetzen. Diese Werkstoffe besitzen neben der erfahrungsgemäß besseren Langzeithaltbarkeit aufgrund noch kleinerer korrosiver Abtragsraten gegenüber den Edelstählen darüber hinaus auch erhebliche Reserven im Falle von unplanmäßigem Auftreten von Wasser als korrosionsförderndem Agens. Die Verwendung dieser Werkstoffe erlaubt vorteilhafte Notbetriebseigenschaften ohne zu befürchtenden schnellen Totalverlust von Apparaten. Wasser kann abweichend vom Regelbetrieb z. B. durch temporäre ungewollte Einschleppung in das System anwesend sein, beispielsweise durch wasserbelastete Einsatz- oder Hilfsstoffe, durch eine Leckage in der Reaktorkühlung oder in den zur fraktionierten Kondensation verwendeten Kondensatoren, oder aufgrund einer Dampfleckage in den Prozess an den direkt mit Dampf beheizten Apparaten.

Zur Reinigung des Reaktors wird der entleerte Reaktor bevorzugt mit auf ca. 80 °C erwärmter Natriumhydroxid-Lösung (z. B. 5 Gew.-% in vollentsalztem Wasser) befüllt und die Lösung im Reaktor umgewälzt. Die nach der Reinigung verbleibende, abgekühlte Lauge wird verworfen, gegebenenfalls nach einer geeigneten Behandlung zur Abgabe in eine Abwasserbehandlungseinrichtung (z. B. einer Kläranlage). Nach der Reinigung des Reaktors, insbesondere von organischen Verschmutzungen, können Reste der Lösung im Reaktorsystem bzw. weiteren gereinigten Anlagenteilen mittels Spülung mit Wasser entfernt werden.

Isobuten ist hochentzündlich und kann im Beisein von Sauerstoff explosive Gemische bilden, die sich in Gegenwart bestimmter Sauerstoffkonzentrationen an heißen Oberflächen entzünden können. Die Anlage wird im Regelbetrieb sowie bei An- und Abfahrvorgängen in geeigneter Weise so betrieben, dass die Sauerstoff-Konzentration in der Gasphase zu jedem Zeitpunkt unterhalb der für eine Explosion erforderlichen Sauerstoffkonzentration liegt. Hierzu wird die Anlage vor der Inbetriebnahme bevorzugt mit einem sauerstoffhaltigen Gas mit einem Sauerstoffgehalt von 10 Vol.-% Sauerstoff oder weniger, vorzugsweise 6 Vol.-% Sauerstoff oder weniger, im Gemisch mit einem Inertgas, vorzugsweise Stickstoff, durchspült und befüllt. Vorzugsweise handelt es sich bei dem sauerstoffhaltigen Gas um sogenannte Magerluft mit einem Sauerstoffgehalt von 10 Vol.-% Sauerstoff oder weniger, hergestellt z. B. durch geeignete Verdünnung von Luft mit z. B. molekularem Stickstoff. Alle dem Prozess zuzuführenden Komponenten werden bevorzugt unter einer Magerluft-Atmosphäre zugeführt. Ein völliger Sauerstoffausschluss ist insbesondere dann nicht erwünscht, wenn einer der nachstehend erläuterten Stabilisatoren Sauerstoff zur Entfaltung seiner Wirksamkeit benötigt. Wird Sauerstoff während des Verfahrens verbraucht, so wird an geeigneten Stellen bevorzugt kontinuierlich frische Magerluft eingespeist, z. B. in den Sumpf der Destillationsvorrichtung. Die Verwendung von Magerluft vermeidet, dass auch bei Inhomogenitäten in der Zusammensetzung der Gasphase ein explosionsgefährlicher Bereich der Gaszusammensetzung durchschritten wird.

Um Leckagen von Luft, insbesondere in unter Unterdruck betriebene Anlagenkomponenten hinein, zu detektieren, sind bevorzugt an verschiedenen Stellen der Anlage online Sauerstoffmessungen installiert. Besonders bevorzugt werden diese online Sauerstoffmessungen in den Leitungen der nicht kondensierbaren Brüden der fraktionierenden Kondensationen installiert.

Der Reaktor ist vollständig mit Flüssigkeit befüllt und wird daher bevorzugt mit einem Sicherheitsventil gegen thermische Ausdehnung abgesichert. Außerdem verfügt der Reaktor bevorzugt über ein Schnellabschott-, Entleer- und Entspannungssystem (SAEES), durch das im Falle einer Leckage der gesamte Reaktorinhalt ohne Kontakt zur Umwelt in einen belüfteten Auffangbehälter abgelassen werden kann, welcher explosionstechnisch sicher beatmet bzw. entlüftet ist. Der Inhalt dieses Auffangbehälters kann bevorzugt über einen Wärmetauscher gekühlt werden, um ggf. durch Nachreaktion entstandene Wärme kontrolliert abführen zu können. Der Auffangbehälter und seine zugeordneten Einrichtungen sind derart gestaltet, dass sein Inhalt in bevorzugter Weise dem Prozess an verschiedenen Stellen wieder zugeführt werden kann.

Die im vorliegenden Verfahren verwendeten Carbonsäuren können, wenn es sich um Carbonsäuren mit ethylenisch ungesättigten Gruppen handelt, vor allem bei höherer Temperatur eine starke Polymerisationsneigung aufweisen. Insbesondere bei Destillationen sind diese Verbindungen im Allgemeinen Temperaturen ausgesetzt, die leicht eine unerwünschte radikalische Polymerisation auslösen können. Dies hat zum einen die Verschmutzung der Apparaturen, das Verstopfen von Leitungen und Pumpen und die Belegung von Kolonnenböden und Wärmeaustauscherflächen zur Folge. Das Reinigen der Anlagen ist ein aufwendiger, teurer und umweltbelastender Vorgang, und die Verfügbarkeit der Anlagen wird dadurch stark reduziert. Zum anderen können unkontrollierte radikalische Polymerisationen ein Sicherheitsrisiko darstellen. Der Einsatz geeigneter Stabilisatoren kann derartige Polymerisationen unterbinden.

Zur Polymerisationsinhibition werden üblicherweise Stabilisatoren bzw. Inhibitoren verwendet. Diese Stabilisatoren sind typischerweise Feststoffe und werden dem Verfahren in Lösung zugeführt. Das Herstellen der Stabilisatorlösungen erfolgt bevorzugt diskontinuierlich als Batch-Ansatz.

Geeignete Stabilisatoren sind beispielsweise N-Oxyl-Verbindungen, Nitrosoverbindungen, Phenolverbindungen, Phenothiazine oder Gemische davon. Die polymerisationsinhibierende Wirkung der Stabilisatoren wird in der Regel durch die Gegenwart von molekularem Sauerstoff verstärkt. In einigen Fällen ist die Anwesenheit von molekularem Sauerstoff für die effektive Wirksamkeit des Stabilisators zwingend erforderlich. Es ist daher bevorzugt, dass in der Anlage molekularer Sauerstoff vorhanden ist.

Geeignete N-Oxyl-Verbindungen umfassen 1-Oxyl-2,2,6,6-tetramethylpiperidin (TEMPO), 1-Oxyl-2,2,6,6-tetramethylpiperidin-4-ol (4-HT), 1-oxyl-2,2,6,6-tetramethylpiperidin-4-on, 1-Oxyl-2,2,6,6-tetramethyl-4-n-propoxypiperidin, 1-Oxyl-2,2,6,6-tetramethyl-4-(2-methoxyethoxy)piperidin, 1-Oxyl-2,2,6,6-tetramethyl-4-(2-methoxyethoxy-acetoxy)piperidin; 1-Oxyl-2,2,6,6-tetramethylpiperidin-4-yl-stearat, 1-Oxyl-2,2,6,6-tetramethylpiperidin-4-yl-acetat, 1-Oxyl-2,2,6,6-tetramethylpiperidin-4-yl-butyrat, 1-Oxyl-2,2,6,6-tetramethylpiperidin-4-yl 2-ethylhexanoat, 1-Oxyl-2,2,6,6-tetramethylpiperidin-4-yl-octanoat, 1-Oxyl-2,2,6,6-tetramethylpiperidin-4-yl-laurat, 1-Oxyl-2,2,6,6-tetramethylpiperidin-4-yl-benzoat, 1-Oxyl-2,2,6,6-tetramethylpiperidin-4-yl-4-tert-butylbenzoat, Bis(1-oxyl-2,2,6,6-tetramethylpiperidin-4-yl)-succinat, Bis(1-oxyl-2,2,6,6-tetramethylpiperidin-4-yl)-adipat, Bis(1-oxyl-2,2,6,6-tetramethylpiperidin-4-yl)-sebacat, Bis(1-oxyl-2,2,6,6-tetramethylpiperidin-4-yl)-n-butylmalonat, Bis(1-oxyl-2,2,6,6-tetramethylpiperidin-4-yl)phthalat, Bis(1-oxyl-2,2,6,6-tetramethylpiperidin-4-yl)-isophthalat, Bis(1-oxyl-2,2,6,6-tetramethylpiperidin-4-yl)-terephthalat, Bis(1-oxyl-2,2,6,6-tetramethylpiperidin-4-yl)-hexahydroterephthalat, 1-Oxyl-2,2,6,6-tetramethyl-4-allyloxy-piperidin, 1-Oxyl-2,2,6,6-tetramethyl-4-acetamidopiperidin, 1-Oxyl-2,2,6,6-tetramethyl-4-(N-butyl-formamido)piperidin, N,N'-Bis(1-oxyl-2,2,6,6-tetramethylpiperidin-4-yl)adipamid, N-(1-Oxyl-2,2,6,6-tetramethylpiperidin-4-yl)-caprolactam, N-(1-Oxyl-2,2,6,6-tetramethyl-piperidin-4-yl)-dodecylsuccinimid, 2,4,6-Tris-(N-butyl-N-(1-oxyl-2,2,6,6-tetramethylpiperidin-4-yl]-s-triazin, 4,4'-Ethylenbis(1-oxyl-2,2,6,6-tetramethylpiperazin-3-one), 1-Oxyl-2,2,6,6-tetramethyl-4-(2,3-dihydroxypropoxy)piperidin, 1-Oxyl-2,2,6,6-tetramethyl-4-(2-hydroxy-4-oxapentoxy)piperidin, Di-tert-butylnitroxyl und 4,4',4"-Tris-(2,2,6,6-tetramethylpiperidinooxyl)phosphit.

1-Oxyl-2,2,6,6-tetramethylpiperidin-4-ol (4-HT) ist besonders geeignet.

Geeignete Nitrosoverbindungen umfassen Nitrosophenol, N-Nitrosodiphenylamin, Isoamylnitrit, N-Nitrosocyclohexylhydroxylamin, N-Nitroso-N-phenylhydroxylamin und deren Salze.

Geeignete Phenolverbindungen umfassen Hydrochinon, 2-Methoxyphenol, 3-Methoxyphenol, 4-Methoxyphenol (MEHQ), 2-Ethoxyphenol, 3-Ethoxyphenol und 4-Ethoxyphenol. 4-Methoxyphenol (MEHQ) ist besonders geeignet.

Geeignete Phenothiazine umfassen Phenothiazin (PTZ), 2-Methylphenothiazin, 2-Octylphenothiazin, 2-Nonylphenothiazin, 2,8-Dimethylphenothiazin, 3,7-Dimethylphenothiazin, 3,7-Diethylphenothiazin, 3,7-Dibutylphenothiazin, 3,7-Dioctylphenothiazin und 2,8-Dioctylphenothiazin, 3,7-Dinonylphenothiazin, 2,8-Dinonylphenothiazin, 2-(α,α-Dimethylbenzyl)phenothiazin, 3,7-Bis(α,αdimethylbenzyl)phenothiazin und 2,8-Bis(α,α-dimethylbenzyl)phenothiazin. Phenothiazin (PTZ) ist besonders geeignet.

Es können auch mehrere Stabilisatoren gleichzeitig zum Einsatz kommen. Die Stabilisatoren kommen im Allgemeinen in Mengen von etwa 2 bis 2000 ppm, bezogen auf die Gesamtmenge an Carbonsäure und Isobuten, zur Anwendung.

Bevorzugter Weise wird der Stabilisator in einem Lösungsmittel gelöst zugegeben. Grundsätzlich geeignet sind alle Lösungsmittel, in denen der jeweilige Stabilisator löslich ist und welches mit der zu stabilisierenden Flüssigphase mischbar ist. Um eine Kontamination mit im Prozess zunächst nicht erforderlichen (externen) Lösungsmitteln bzw. die Erfordernis des Abtrennens eines externen Lösungsmittels zu vermeiden, verwendet man als Lösungsmittel bevorzugt eine im Prozess ohnehin vorhandene Flüssigkeit. Besonders bevorzugt wird der reine Zielester als Lösungsmittel verwendet.

Das Einbringen des Stabilisators erfolgt jeweils in üblicher Weise durch mengenkontrollierte Zufuhr mittels Pumpen, vorzugsweise wird die Stabilisatorlösung zur besseren Verteilung mittels Sprühvorrichtungen wie Spraydüsen eingesprüht.

Einige der genannten Stabilisatoren sind nur in Anwesenheit von Sauerstoff wirksam, beispielsweise MEHQ, wodurch eine relativ hohe Sauerstoffkonzentration, wie sie beispielsweise in Luft vorliegt, vorteilhaft wäre. Andererseits soll die Sauerstoffkonzentration auf verhältnismäßig niedrige Werte begrenzt sein, damit keine explosionsfähigen Gemische auftreten. Das Verfahren wird geeigneter Weise so durchgeführt, dass die Sauerstoff-Konzentration in der Gasphase an allen relevanten Stellen und zu jedem Zeitpunkt unterhalb der Explosionsgrenze liegt. Bevorzugt liegt das Volumenverhältnis von Sauerstoff zu Stickstoff in allen gasförmigen Gemischen, die in den Stufen a) bis d) auftreten, im Bereich von 0,03 bis 0,11.

Die Polymerisationsneigung besteht vor allem in der flüssigen Phase bei verminderten Konzentrationen an Stabilisatoren und ggf. Sauerstoff. Da die Stabilisatoren in der Regel schwerflüchtig sind, sammeln sie sich bei Verdampfungsschritten im Sumpf der jeweiligen Verdampfungsanlage. Es ist daher normalerweise nötig, nach der Verdampfung von polymerisationsfähigen Verbindungen erneut Stabilisator zuzugeben, wenn die Verbindungen kondensiert werden, da das Kondensat in der Regel weitestgehend frei von Stabilisatoren anfällt.

Das erfindungsgemäße Verfahren umfasst eine Vielzahl an Verfahrensschritten, bei denen Stoffgemische mit sehr unterschiedlichen Zusammensetzungen bei verschiedensten Prozessbedingungen vorliegen. Zur Sicherstellung eines sicheren und wirtschaftlich vorteilhaften Betriebs ist eine Variation der jeweils zugesetzten Stabilisatoren notwendig, welche an verschiedenen Stellen ins Verfahren eingeführt werden.

In einer bevorzugten Ausführungsform ist bei der Umsetzung der Carbonsäure mit Isobuten ein unter Phenothiazinen ausgewählter Stabilisator, besonders bevorzugt PTZ, zugegen. Die eingesetzte (Meth)acrylsäure kann bereits mit PTZ vorstabilisiert werden, was insbesondere bei der Inbetriebnahme der Anlage von Vorteil ist. Weitere Mengen PTZ können in den Reaktor dosiert werden. PTZ geht bei der Abtrennung des sauren Katalysators mittels der partiellen Verdampfung des Veresterungsgemisches in die flüssige Schwersiederphase über, welche vom produkthaltigen Hauptstrom abgetrennt wird. Die flüssige Schwersiederphase wird vorzugsweise wieder in den Reaktor zurückgeführt, sodass im Allgemeinen nur geringe Ergänzungsmengen an PTZ dem Prozess kontinuierlich frisch zugeführt werden müssen.

In einer bevorzugten Ausführungsform wird ein unter N-Oxyl-Verbindungen ausgewählter Stabilisator bei den Kondensationsschritten zugesetzt. Besonders bevorzugt wird eine Lösung von 4-HT in Zielester zugegeben. Die Zugabe des Stabilisators erfolgt vorzugsweise am Brüdeneintritt in den Kondensator, bei einer zweistufigen Partialkondensation am Brüdeneintritt in den ersten Kondensator. Mit einem Rückführstrom des Kondensats zum Brüdeneintritt des zweiten Kondensators gelangt dieser Stabilisator auch in den zweiten Kondensator.

Die Leichtsieder-Destillationskolonne wird ebenfalls bevorzugt mit einem unter N-Oxyl-Verbindungen ausgewählten Stabilisator, besonders bevorzugt 4-HT, stabilisiert. Der Zulaufstrom in die Leichtsieder-Destillationskolonne enthält 4-HT aus dem vorhergehenden Schritt, eine weitere Menge 4-HT wird am Kopf der Leichtsieder-Destillationskolonne zugegeben, insbesondere am Brüdeneintritt des ersten Kondensators und gelangt mit dem Kondensatrücklauf auch in die Leichtsieder-Destillationskolonne.

In einer bevorzugten Ausführungsform wird ein unter N-Oxyl-Verbindungen ausgewählter Stabilisator, besonders bevorzugt 4-HT, dem Zulauf zur Destillationsvorrichtung zugesetzt.

Der Sumpf und Abtriebsteil der Destillationsvorrichtung werden durch die N-OxylVerbindung stabilisiert. Es ist bevorzugt, den Verstärkungsteil der Destillationsvorrichtung nicht mit N-Oxyl-Verbindungen zu stabilisieren, da der Übertritt derartiger Verbindungen nicht vollständig vermeidbar wäre. Die N-Oxyl-Verbindungen sind im Zielester nicht erwünscht, weil sie zu Verfärbungen des Produkts und daraus hergestellten Substanzen führen können. Daher wird im Verstärkungsteil der Destillationsvorrichtung ein unter Phenolverbindungen ausgewählter Stabilisator, insbesondere MEHQ, zugesetzt. Dieser Stabilisator wird auch zur Stabilisierung des Produkts verwendet und wirkt daher nicht nachteilig bzw. muss nicht in einem späteren Schritt abgetrennt werden. MEHQ wird bevorzugt dem Umwälzstrom über die Kondensatoren und/oder dem Kondensatrücklauf in die Destillationsvorrichtung zugesetzt. Zweckmäßigerweise erfolgt dies durch Eindüsen über eine zentrisch im Brüdenrohrausgang eingebaute Düse.

Um die Wirksamkeit des MEHQ zu gewährleisten, wird in den Sumpf der Destillationsvorrichtung bevorzugt ein molekularen Sauerstoff enthaltendes Gas, bevorzugt Magerluft (5 Vol.-% Sauerstoff in Stickstoff) eingespeist. Durch diese Maßnahmen ist es möglich, die Polymerisatbildung in den Kondensatoren, den Brüdenrohren und in der Destillationsvorrichtung zu verhindern oder zumindest so weit zu unterdrücken, dass wirtschaftlich vorteilhaft lange Laufzeiten des Betriebs ohne Reinigungsabstellung möglich sind.

Die Erfindung wird durch die beigefügten Figuren und die Beispiele näher veranschaulicht.
Fig. 1 ist eine schematische Darstellung einer zur Durchführung des erfindungsgemäßen Verfahrens geeigneten Anlage.
Fig. 2 ist eine schematische Darstellung einer zur Durchführung der Reindestillation geeigneten Destillationsvorrichtung.
Fig. 3 zeigt den Differenzdruck in einer Destillationsvorrichtung gemäß Fig. 2, welche mit einem Naturumlaufverdampfer und ohne Beheizung des Kopfbereichs der Destillationsvorrichtung betrieben wird, in Abhängigkeit von der Zeit.

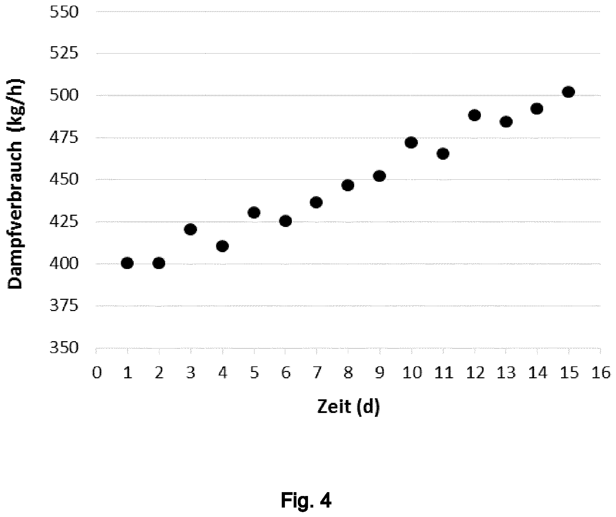

Fig. 4 zeigt die Heizleistung einer Destillationsvorrichtung gemäß Fig. 2, welche mit einem Naturumlaufverdampfer und mit Beheizung des Kopfbereichs der Destillationsvorrichtung betrieben wird, als Dampfverbrauch in Abhängigkeit von der Zeit.

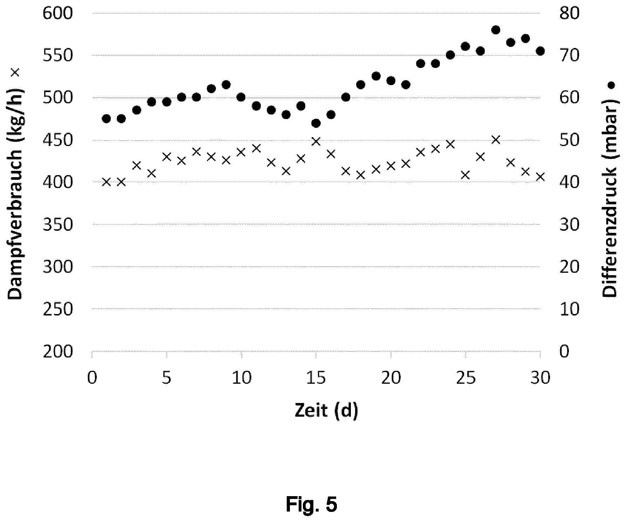

Fig. 5 zeigt den Differenzdruck und die Heizleistung (als Dampfverbrauch) in einer Destillationsvorrichtung gemäß Fig. 2, welche mit einem Zwangsumlaufverdampfer und mit Beheizung des Kopfbereichs der Destillationsvorrichtung betrieben wird, in Abhängigkeit von der Zeit.

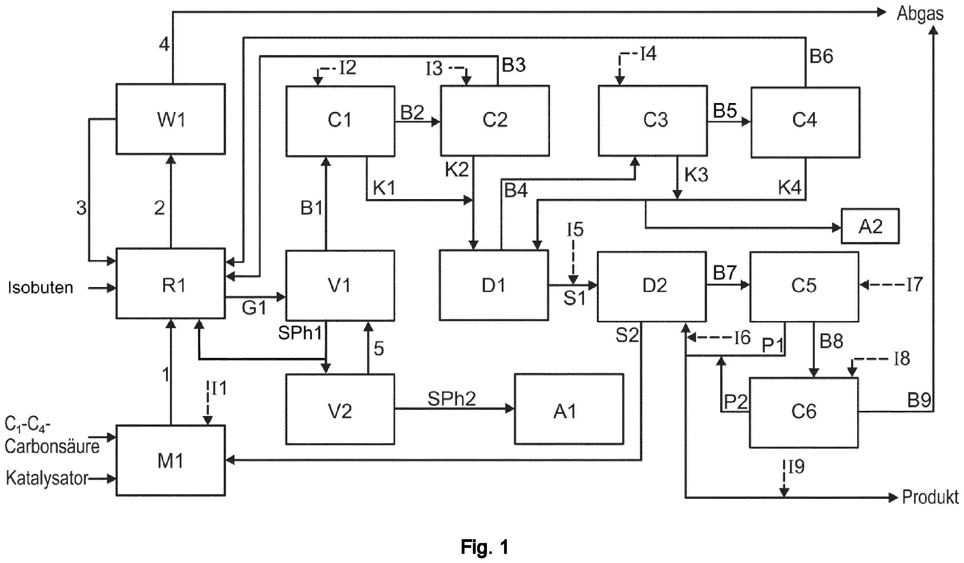

Gemäß Fig. 1 werden über einen Mischer M1 eine ethylenisch ungesättigte C₁-C₄-Carbonsäure, ein Stabilisator I1 und der saure Katalysator als Gemisch dem Reaktor R1 über eine Leitung 1 und eine Düse E1 (in Fig. 1 nicht dargestellt) zugeführt. Isobuten wird in den Sumpf des Reaktors R1 eingeführt. Über die Düse E1 werden dem Reaktor R1 außerdem die Isobuten-haltigen nicht kondensierten Brüden B3 und B6 der Kondensatoren C2 und C4 zugeführt. Das Kondensat des Rücklaufkühlers W1 wird dem Reaktor R1 zugeführt.

Im Reaktor R1 findet die Additionsreaktion von Isobuten und der ethylenisch ungesättigten C₁-C₄-Carbonsäure statt. Der Reaktor hat vier gekühlte Reaktionszonen. Die Reaktionszonen sind durch Trennbleche voneinander getrennt, wobei der Übergang von einer Reaktionszone zur nächsten aus einem Loch mit geringem Querschnitt besteht. Die Durchmischung der Reaktanden im Reaktor erfolgt durch die Düse E1 und durch Verwirbelung beim Übergang von einer Zone zur nächsten.

Der Abzug des flüssigen Reaktionsprodukts G1 am Kopf des Reaktors R1 erfolgt mittels einer Standregelung, so dass sich eine konstante Flüssig/Gas-Phasengrenze eingestellt. Die im Wesentlichen aus Inertgasen, Isobuten und geringen Mengen des tert-Butylester bestehende Gasphase wird über die Leitung 2 dem Rücklaufkühler W1 zugeführt. Das Kondensat des Rücklaufkühlers W1 enthält Isobuten und Acrylsäure und wird über die Leitung 3 dem Reaktor R1 zugeführt. Die Gasphase des Rücklaufkühlers W1 wird über die Leitung 4 als Abgas aus dem Verfahren ausgeführt.

Das flüssige Reaktionsprodukt G1 wird seitlich am Kopf des Reaktors R1 abgezogen und mengengeregelt der Verdampfungseinheit V1, bestehend aus einem Fallfilmverdampfer und einem Abscheidebehälter (in Fig.1 nicht einzeln dargestellt), zugeführt. Der Druck des flüssigen Reaktionsprodukts wird mittels eines Drosselventils (in Fig. 1 nicht dargestellt) von Reaktordruck auf Unterdruck abgesenkt, bei welchem die folgende Katalysatorabtrennung erfolgt. Im Fallfilmverdampfer der Verdampfungseinheit V1 wird das Reaktionsgemisch partiell verdampft und in den Abscheidebehälter weitergeführt. Der Abscheidebehälter enthält bevorzugt einen Tropfenabscheider, um mitgerissene Schwersiederkomponenten wie Schwefelsäure und den Stabilisator I1 sicher abzutrennen. Die nicht gasförmigen Bestandteile werden im Abscheidebehälter als erste Schwersiederphase SPh1 gesammelt und über einen externen Kühler (in Fig. 1 nicht abgebildet) gekühlt, um eine Rückreaktion des darin enthaltenen tert-Butylesters zur Carbonsäure und Isobuten zu verhindern.

Ein Teil der ersten Schwersiederphase SPh1 wird mengengeregelt dem Dünnschichtverdampfer V2 zugeführt, um die weitere Abtrennung von Carbonsäure bzw. tert-Butylester als Gas zu ermöglichen. Die im Dünnschichtverdampfer V2 erzeugte Gasphase wird über die Leitung 5 in den Abscheidebehälter der Verdampfungseinheit V1 zurückgeführt, während ein Teil der flüssigen zweiten Schwersiederphase SPh2 in den Absetzbehälter A1 geführt wird. Bevorzugt werden Teilströme der zweiten Schwersiederphase SPh2 dazu verwendet, um den Zufuhrstrom zum Dünnschichtverdampfer der Verdampfungseinheit V1 vorzuwärmen. Durch Variation der heißen Teilströme lässt sich die Zusammensetzung des Zufuhrstroms zum Dünnschichtverdampfer der Verdampfungseinheit V1 sowie die Temperatur des Zufuhrstroms variieren.

Ein weiterer Teil der ersten Schwersiederphase SPh1 und ein weiterer Teil der zweiten Schwersiederphase SPh2 werden miteinander oder jeweils einzeln mengengeregelt über die Düse E1 in den Reaktor R1 zurückgeführt (die Rückführung der zweiten Schwersiederphase SPh2 ist in Fig. 1 nicht abgebildet).

Die gasförmigen Bestandteile aus dem Abscheidebehälter der Verdampfungseinheit V1 werden in den Kondensatoren C1 und C2 fraktionierend kondensiert, wobei der Brüden B2 des Kondensators C1 in den Kondensator C2 geführt wird. Ein Stabilisator I2 wird am Kopf des Kondensators C1 zugegeben und ein Stabilisator I3 am Kopf des Kondensators C2 zugegeben. Für die Kühlung des Kondensators C1 kann z. B. Flusswasser oder gleichwertig temperiertes Kühlwasser verwendet werden, während der Kondensator C2 mit Solekühlung betrieben wird. Der im Kondensator C2 nicht kondensierte Brüden B3 wird über die Düse E1 in den Reaktor R1 geführt.

Die in den Kondensatoren C1 und C2 anfallenden Kondensate K1 und K2 werden vereinigt und der Destillationskolonne D1 seitlich zugeführt. In der Destillationskolonne D1 werden Leichtsieder, vor allem Diisobuten und Isobuten, abgetrennt. Die Sumpfbeheizung der Destillationskolonne D1 erfolgt über einen Umlaufverdampfer (in Fig. 1 nicht dargestellt), über den ein Teil des Sumpfs umgepumpt wird. Die Leichtsieder B4 werden dampfförmig am Kopf der Destillationskolonne D1 abgeführt und in den Kondensatoren C3 und C4 fraktionierend kondensiert. Der Brüden B5 des Kondensators C3 wird in den Kondensator C4 geführt. Für die Kühlung des Kondensators C3 kann z. B. Flusswasser oder gleichwertig temperiertes Kühlwasser verwendet werden, während der Kondensator C4 mit Solekühlung betrieben wird. Der im Kondensator C4 nicht kondensierte Brüden B6 wird über die Düse E1 in den Reaktor R1 geführt. Ein Stabilisator I4 wird am Kopf des Kondensators C3 zugegeben. Die in den Kondensatoren C3 und C4 anfallenden Kondensate K3 und K4 werden vereinigt; ein Teilstrom wird als Rücklauf in die Destillationskolonne D1 geführt, der Rest wird dem Absetzbehälter A2 zugeführt.

Der Sumpfstrom S1 der Destillationskolonne D1 wird der Destillationskolonne D2 seitlich zugeführt. In den Zulauf zur Destillationskolonne D2 wird Stabilisator I5 dosiert. Die Sumpfbeheizung der Destillationskolonne D2 erfolgt über einen Umlaufverdampfer (in Fig. 1 nicht dargestellt), über den ein Teil des Sumpfs umgepumpt wird. Im Rahmen des Umpumpens wird dem Sumpf der Destillationskolonne D2 außerdem Magerluft zugeführt.

In der Destillationskolonne D2 wird der tert-Butylester von der verbleibenden ethylenisch ungesättigten Carbonsäure getrennt. Üblicherweise liegt der Siedepunkt der Carbonsäure über dem Siedepunkt des tert-Butylesters, weshalb der reine tert-Butylester über Kopf abgezogen wird und die Carbonsäure am Sumpf der Destillationskolonne D2 anfällt. Um eine Kondensation des tert-Butylesters am Kolonnenkopf zu vermeiden, wird der Kolonnenkopf mit Dampf beheizt. Somit wird auch einer aus der Kondensation ggf. resultierenden Polymerisation des tert-Butylesters vorgebeugt. Der Sumpfstrom S2 der Destillationskolonne D2 wird über einen Wärmetauscher (in Fig. 1 nicht dargestellt) und ggf. einen Filter (in Fig. 1 nicht dargestellt) in den Reaktor R1 zurückgeführt. Der Filter kann mit einer Bypassleitung versehen sein, um diesen zur Reinigung oder zum Austausch ohne Unterbrechung des Verfahrens entnehmen zu können.

Der Brüden B7 der Destillationskolonne D2 wird in den Kondensatoren C5 und C6 fraktionierend kondensiert; der Brüden B8 des Kondensators C5 wird in den Kondensator C6 geführt. Ein Stabilisator I7 wird am Kopf des Kondensators C5 zugegeben und ein Stabilisator I8 am Kopf des Kondensators C6 zugegeben. Der im Kondensator C6 nicht kondensierte Brüden B9 wird als Abgas aus dem Verfahren ausgeführt. Das Abgas wird zum Beispiel einer Fackel oder einer Abgasverbrennung zugeführt.

Ein Teilstrom der vereinigten Kondensate P1 und P2 aus den Kondensatoren C5 und C6 wird auf die Kondensatoren C5 und C6 (in Fig. 1 nicht abgebildet) bzw. unter Zusatz des Stabilisators I6 als Rücklauf auf die Destillationskolonne D2 gegeben. Ein weiterer Teilstrom der vereinigten Kondensate P1 und P2 aus den Kondensatoren C5 und C6 wird als reiner tert-Butylester über einen Wärmetauscher (in Fig. 1 nicht abgebildet) aus dem Verfahren ausgeführt. Zur Lagerstabilisierung kann dem reinen tert-Butylester weiterer Stabilisator I9 zugegeben werden.

Die Anlage verfügt bevorzugt über ein Schnellabschott-, Entleer- und Entspannungssystem (SAEES), durch das im Falle eines Lecks der gesamte Inhalt des Reaktors R1 in einen belüfteten Auffangbehälter (in Fig. 1 nicht dargestellt) abgelassen werden kann. Der Inhalt dieses Auffangbehälters kann über einen Wärmetauscher gekühlt werden, um die durch Nachreaktion entstandene Wärme abführen zu können. Der Inhalt des Auffangbehälters kann dem Verfahren an verschiedenen Stellen wieder zugeführt werden, insbesondere dem Reaktor R1, dem Fallfilmverdampfer V2 oder dem Dünnschichtverdampfer der Verdampfungseinheit V1.

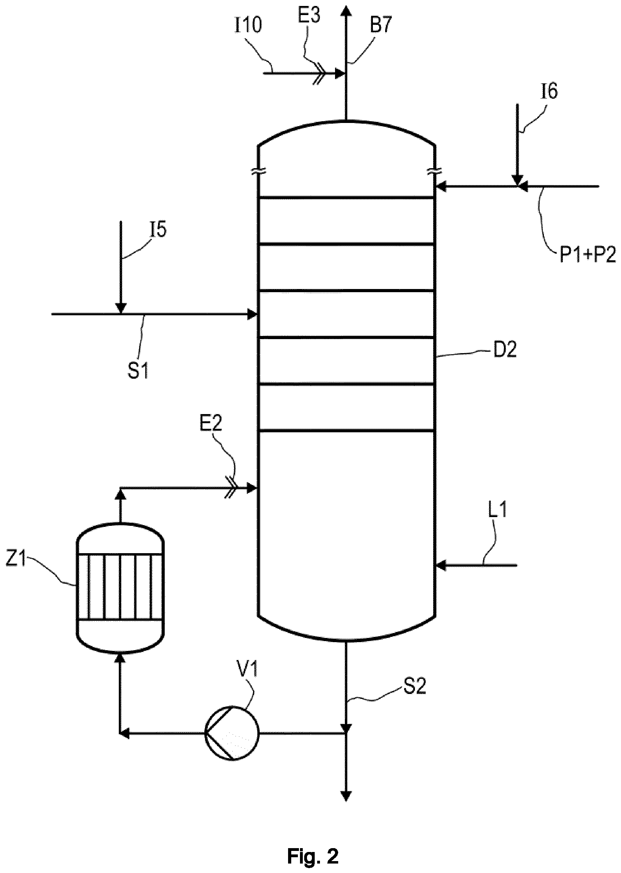

Fig. 2 ist eine bevorzugte Ausführungsform von Fig. 1. Gemäß Fig. 2 wird der Sumpfstrom S1 der Destillationskolonne D1 der Destillationskolonne D2 seitlich zugeführt. In den Zulauf zur Destillationskolonne D2 wird Stabilisator I5 dosiert. Ein Teilstrom der vereinigten Kondensate P1 und P2 aus den Kondensatoren C5 und C6 (in Fig. 2 nicht abgebildet) wird unter Zusatz des Stabilisators I6 als Rücklauf auf die Destillationskolonne D2 gegeben.

Die Sumpfbeheizung der Destillationskolonne D2 erfolgt über einen Zwangsumlaufverdampfer Z1, über den ein Teil des Sumpfs S2 mittels der Pumpe V1 umgepumpt wird. Der erhitzte Strom aus dem Umlaufverdampfer wird über eine Düse E2 in die Destillationskolonne D2 zurückgeführt. Dem Sumpf der Destillationskolonne D2 wird Magerluft L1 zugeführt.

In der Destillationskolonne D2 wird der tert-Butylester von der verbleibenden ethylenisch ungesättigten Carbonsäure getrennt. Üblicherweise liegt der Siedepunkt der Carbonsäure über dem Siedepunkt des tert-Butylesters, weshalb der reine tert-Butylester über Kopf abgezogen wird und die Carbonsäure am Sumpf der Destillationskolonne D2 anfällt. Um eine Kondensation des tert-Butylesters am Kolonnenkopf zu vermeiden, wird der Kolonnenkopf mit Dampf beheizt. Somit wird auch einer aus der Kondensation ggf. resultierenden Polymerisation des tert-Butylesters vorgebeugt. Dieser Polymerisation wird weiterhin durch die Dosierung eines Inhibitors I10 in das Brüdenrohr B7 mittels einer Düse D3 vorgebeugt.

### Beispiel 1

Das folgende Beispiel wurde in einer Anlage gemäß Fig. 1 durchgeführt. Alle angegebenen Prozentsätze sind gewichtsbezogen, soweit nicht anders angegeben. Es wurden Methacrylsäure und Isobuten unter Zusatz von Schwefelsäure zu tert-Butylmethacrylat umgesetzt. Die Anlage wurde mit Magerluft (5 Vol.-% Sauerstoff in Stickstoff) durchspült und befüllt.

Methacrylsäure (MAS, 99,97%, 0,012% Essigsäure, 0,011% Propionsäure, 575 kg/h) wurde in einem Mischer M1 mit der aus dem Sumpf der Destillationskolonne D2 rückgeführten Methacrylsäure (91,35% MAS, 5,15% Schwersieder, 3.32% TBMA, 485 kg/h) und mit einer Phenothiazin (PTZ)-Lösung (97,97% MAS, 2,10% PTZ, 0,01 m³/h) als Stabilisator I1 vermischt. Schwefelsäure (technisch, 96%, 3,7 kg/h) wurde mittels Stickstoff (3,5 bar abs.) eindosiert. Außerdem wurde ein Teilstrom (1.500 kg/h) der flüssigen Phase aus dem Abscheidebehälter der Verdampfungseinheit V1 dem Gemisch zugeführt.

Die flüssigen Edukte und Rückströme wurden als Gemisch über die Leitung 1, einen Kühler (Rohrbündel, 180 m², Edelstahl 1.4571, in Fig.1 nicht dargestellt) und die Düse E1 dem Reaktor R1, einer kaskadierten Blasensäule, zugeführt. Die Austrittstemperatur des Kühlers betrug 33 °C.

Isobuten (402 kg/h) wurde direkt in den Sumpf des Reaktors dosiert. Außerdem wurden die Isobuten-haltigen Brüden B3 und B6 aus den Kondensatoren C2 und C4 (122 m³/h), zusammen mit dem tert-Butylmethacrylat-haltigen Kondensat aus dem Rücklaufkühler W1, über die Düse E1 in den Reaktor R1 dosiert. Bei der Düse E1 handelte es sich um eine Ejektorstrahldüse. In der Düse erfolgt eine Druckerhöhung durch den Treibstrahl auf ca. 2 bar abs.

Der Reaktor R1 hatte vier Reaktionszonen, wobei der Übergang aus einem Loch mit geringem Querschnitt (Durchmesser 24 mm) bestand. Die Reaktionszonen wurden jeweils gekühlt (Zone 1: externer Flusswasserkühler mit 121 kW, Zone 2: externer Solekühler mit 28 kW, Zone 3: innenliegender Solekühler mit 14 kW, Zone 4: innenliegender Solekühler mit 14 kW, wobei die Temperatur der Sole je -20 °C betrug). Im Reaktor R1 fand die leicht exotherme Additionsreaktion (-37,6 kJ/mol) von Isobuten und Methacrylsäure bei einer Temperatur von 35 °C in der Zone 1, 25 °C in der Zone 2, 20 °C in der Zone 3 und 16 °C in der Zone 4 und einem Druck von 1,92 bar abs. statt.

Die Durchmischung der Reaktanden im Reaktor erfolgte zum einen durch die Düse E1 und zum anderen durch Verwirbelung beim Übergang von einer Zone zur Nächsten. Am Kopf des Reaktors (Zone 4) wurde mittels Standregelung eine Flüssig/Gas-Phasengrenze eingestellt.

Die Gasphase umfasste 41,18% Isobuten, 0,74% tert-Butylmethacrylat (TBMA) und Inertgase und wurde über die Leitung 2 in den Rücklaufkühler W1 geführt. Das im Abgas mitgerissene TBMA wurde mittels Rücklaufkühler W1 auskondensiert und über die Leitung 3 im Gemisch mit den Isobuten-haltigen Brüden aus den Kondensatoren C2 und C4 über die Düse E1 in den Reaktor R1 zurückgeführt. Der Druck am Kopf des Reaktors R1 wurde in der Abgasleitung auf 1,21 bar abs. eingestellt. Am Boden des Reaktors stellte sich ein Druck von 1,92 bar abs. ein. Die gasförmigen Bestandteile des Rücklaufkühlers W1 (4,37 m³/h) wurden über die Leitung 4 aus dem Verfahren ausgeführt.

Das flüssige Reaktionsprodukt aus dem Reaktor R1 hatte folgende Zusammensetzung:

| | |
|---|---|
| 8,58% | Isobuten |
| 41,63% | MAS |
| 44,00% | TBMA |
| 0,68% | Diisobuten |
| 2,66% | Schwersieder |
| 2,10% | Schwefelsäure |
| 0,35% | sonstige Bestandteile |

Das flüssige Reaktionsprodukt aus dem Reaktor R1 (3,39 m³/h, 16 °C) wurde am oberen Ende des Reaktors R1 abgeführt und über einen Siebkorbfilter (0,1 m², in Fig. 1 nicht abgebildet) dem Fallfilmverdampfer (71,5 °C, 309 kW, 47 m²) der Verdampfungseinheit V1 zugeführt. Über ein Regelventil (Durchflussregelung) wurde der Druck auf 60 mbar abs. reduziert. Es bildete sich ein zweiphasiges Gemisch durch das Verdampfen eines Teils der Leichtsiederkomponenten. Im Fallfilmverdampfer der Verdampfungseinheit V1 wurde das zweiphasige Gemisch temperaturgeregelt bei 55,2 °C und 70 mbar abs. weiter abgedampft und anschließend in den Abscheidebehälter der Verdampfungseinheit V1geleitet. Der Abscheidebehälter war mit einem Tropfenabscheider ausgestattet, um sicher Schwefelsäure und PTZ abzutrennen.

Die nicht gasförmigen Bestandteile im Abscheidebehälter der Verdampfungseinheit V1 wurden als erste Schwersiederphase SPh1 über einen Umpumpstrom mittels eines Solekühlers auf 2 °C gekühlt. Im Abscheidebehälter stellte sich so eine Mischtemperatur von ca. 8 bis 10 °C ein. Ein Teil des Umpumpstroms (1.500 kg/h) der ersten Schwersiederphase SPh1 wurde zur Schwefelsäurerückführung dem Reaktor R1 wieder zugeführt. Darüber hinaus wurde ein Teil des Umpumpstroms der ersten Schwersiederphase SPh1 (82 kg/h) dem Dünnschichtverdampfer V2 (4 m², Nickel-Chrom-Molybdän-Legierung 2.4610) zugeführt, um weitere Wertprodukte (TBMA, MAS) über den Kopf des Dünnschichtverdampfers V2 abzutrennen (89 °C, 70 mbar abs.). Die Beheizung des Dünnschichtverdampfers V2 erfolgte mittels Niederdruck-Dampf. Dem Sumpfaustrag des Dünnschichtverdampfers V2 war eine Pumpe (in Fig.1 nicht dargestellt) nachgeschaltet, welche die auszuschleusende zweite Schwersiederphase SPh2 in einem Teilstrom zum Absetzbehälter A1 leitete. Auf dem Weg zum Absetzbehälter A1 wurde der Teilstrom der zweiten Schwersiederphase SPh2 von 89 °C auf 35 °C gekühlt. Dies erfolgte mittels eines Mantelrohrs, durch das im Gegenstrom Wasser mit einer Temperatur von 30 °C geführt wurde.

Ein weiterer Teilstrom der zweiten Schwersiederphase SPh2 des Dünnschichtverdampfers V2 wurde wiederum als heißer Rückführstrom direkt dem Zufuhrstrom zum Dünnschichtverdampfer V2 zugemischt. Durch Variation des heißen Rückführstroms ließen sich der Zufuhrstrom, sowie auch die Zufuhrstromtemperatur in einem weiten Bereich einstellen. In Verbindung mit der Anpassung der Heizdampfmenge und der Heizdampftemperatur war der Dünnschichtverdampfer V2 in der Lage, einen großen Lastbereich abzudecken.

Noch ein weiterer Teilstrom der zweiten Schwersiederphase SPh2 des Dünnschichtverdampfers wurde dem kalten Umpumpstrom auf der Saugseite der Pumpe zum Dünnschichtverdampfer V2 zugegeben, der sich dadurch aber nur wenig erwärmte. Der Zufuhrstrom zum Dünnschichtverdampfer V2 wurde auf der Druckseite der Pumpe entnommen.

Der Brüden des Dünnschichtverdampfers V2 wurde über die Leitung 5 dem Abscheidebehälter der Verdampfungseinheit V1 zugeführt. Der Brüden B1 aus dem Abscheidebehälter der Verdampfungseinheit V1 (ca. 68 °C) hatte folgende Zusammensetzung:

| | |
|---|---|
| 57% | TBMA |
| 24% | MAS |
| 16% | Isobuten |
| 3% | sonstige Bestandteile |

Der Brüden B1 wurde fraktionierend kondensiert und dazu in den Kopf des Kondensators C1 (Rohrbündelwärmetauscher, 75 m², Kühlung: Flusswasser (27 °C), 60 mbar abs., Edelstahl 1.4571) geleitet. Im Kondensator C1 wurde das zugeführte Gemisch auf 29 °C gekühlt.

Der Brüden B2 des Kondensators C1 (umfassend ca. 35% TBMA, 5% MAS, 60% Isobuten) wurde in den Kopf des Kondensators C2 (Rohrbündelwärmetauscher, 30 m², Kühlung: Kühlsole (-20 °C), 60 mbar abs., Edelstahl 1.4571) geführt. Das Kondensat K2 des Kondensators C2 (umfassend ca. 86% TBMA, 5% MAS, 4% Isobuten, ca. - 17 °C) wurde in einem Behälter (in Fig. 1 nicht dargestellt) mit dem Kondensat K1 des Kondensators C1 vereinigt. Der Brüden B3 des Kondensators C2 (umfassend ca. 95% Isobuten) wurde über eine Pumpe (in Fig. 1 nicht dargestellt) mit dem Brüden B6 des Kondensators C4 gemischt und in den Reaktor R1 zurückgeführt.

Das Kondensat K1 des Kondensators C1 (umfassend ca. 68% TBMA, 28% MAS, 0,7% Isobuten) wurden in einem Behälter (in Fig. 1 nicht dargestellt) mit dem Kondensat K2 des Kondensators C2 vereinigt. Das vereinigte Kondensat aus C1 und C2 hatte folgende Zusammensetzung:

| | |
|---|---|
| 68,09% | TBMA |
| 28,17% | MAS |
| 1,13% | Diisobuten |
| 0,74% | Isobuten |
| 0,61% | sonstige Bestandteile |

Ein Teilstrom der vereinigten Kondensate aus C1 und C2 wurde zusammen mit einer 4-Hydroxy-TEMPO (4-HT)-Lösung (2% in TBMA) als Stabilisator I2 in den Kopf des Kondensators C1 geleitet, wiederum ein Teilstrom davon wurde als Stabilisator I3 in den Kopf des Kondensators C2 geleitet.

Ein weiterer Teilstrom der vereinigten Kondensate aus den Kondensatoren C1 und C2 wurde der Destillationskolonne D1 (40 Dual-Flow-Böden, 91 °C im Kolonnensumpf, 120 mbar abs. im Kolonnenkopf) auf den Boden 23 zugeführt. Die Destillationskolonne D1 wurde über einen Naturumlaufverdampfer (4 bar abs. Dampf) geheizt. Die Temperaturregelung der Destillationskolonne D1 erfolgte über ein Regelventil in der Rücklaufleitung. Das Vakuum wurde mittels Regelventil in der Saugleitung zur Vakuumeinheit geregelt.

Der Brüden B4 aus der Destillationskolonne D1 wurde fraktionierend kondensiert und dazu in den Kondensator C3 (Rohrbündelwärmetauscher, 110 m², Kühlung: Flusswasser (27 °C), 120 mbar abs., Edelstahl 1.4571) geleitet. Im Kondensator C3 wurde das zugeführte Gemisch auf 29 °C gekühlt. Das Kondensat K3 des Kondensators C3 wurde in einem Behälter mit dem Kondensat K4 des Kondensators C4 vereinigt.

Der Brüden B5 des Kondensators C3 wurde in den Kondensator C4 (Rohrbündelwärmetauscher, 8 m², Kühlung: Kühlsole (-20 °C), 120 mbar abs., Edelstahl 1.4571) geleitet und auf -2 °C gekühlt. Das Kondensat K4 des Kondensators C4 wurde in einem Behälter (in Fig. 1 nicht dargestellt) mit dem Kondensat K3 des Kondensators C3 vereinigt. Der Brüden B6 des Kondensators C4 (65,12 m³/h, 62,94% Isobuten) wurde über eine Pumpe (in Fig. 1 nicht dargestellt) mit dem Brüden B3 des Kondensators C2 und dem Kondensat des Rücklaufkühlers W1 gemischt und in den Reaktor R1 zurückgeführt.

Ein Teilstrom der vereinigten Kondensate aus C3 und C4 wurde in den Kopf der Destillationskolonne D1 geleitet; wiederum ein Teilstrom davon wurde als Gemisch mit einer 4-HT-Lösung (2% in TBMA) als Stabilisator I4 in den Kopf des Kondensators C3 geleitet.

Das Sumpfprodukt der Destillationskolonne 1 hatte folgende Zusammensetzung:

| | |
|---|---|
| 69,63% | TBMA |
| 28,65% | MAS |
| 1,72% | sonstige Bestandteile |

Das Sumpfprodukt S1 der Destillationskolonne D1 wurde mit einer 4-HT-Lösung (2% in TBMA) als Stabilisator I5 versetzt und der Destillationskolonne D2 (40 Dual-Flow-Böden, 99 °C im Kolonnensumpf, 60 mbar abs. im Kolonnenkopf) auf den Boden 18 zugeführt. Die Destillationskolonne D2 wurde über einen Umlaufverdampfer (4 bar abs. Dampf) geheizt. Es handelte sich entweder um einen Naturumlaufverdampfer oder einen Zwangsumlaufverdampfer (sh. Varianten 1 bis 3). Die Temperaturregelung der Destillationskolonne D2 erfolgte über ein Regelventil in der Rücklaufleitung. Das Vakuum wurde mittels Regelventil in der Saugleitung zur Vakuumeinheit geregelt.

In den Sumpf der Destillationskolonne D2 wurden 6 m³/h Magerluft (5 Vol.-% Sauerstoff in Stickstoff) dosiert.

Der Brüden B7 aus der Destillationskolonne D2 (umfassend 99,83% TBMA) wurde fraktionierend kondensiert und dazu in den Kondensator C5 (Rohrbündelwärmetauscher, 72 m², Kühlung: Flusswasser (27 °C), 60 mbar abs., Edelstahl 1.4571) geführt. Im Kondensator C5 wurde das zugeführte Gemisch auf 29 °C gekühlt. Das Kondensat P1 des Kondensators C5 wurde in einem Behälter (in Fig. 1 nicht dargestellt) mit dem Kondensat P2 des Kondensators C6 vereinigt.

Der Brüden B8 des Kondensators C5 wurde in den Kopf des Kondensators C6 (Rohrbündelwärmetauscher, 12 m², Kühlung: Kühlsole (-20 °C), 55 mbar abs. Edelstahl 1.4571) geleitet und auf -17 °C gekühlt. Das Kondensat P2 des Kondensators C6 wurde in einem Behälter (in Fig. 1 nicht dargestellt) mit dem Kondensat P1 des Kondensators C5 als Produkt vereinigt. Der Brüden B9 des Kondensators C6 wurde über eine Pumpe (in Fig. 1 nicht dargestellt) aus dem Verfahren ausgetragen.

Ein Teilstrom der vereinigten Kondensate P1 und P2 aus den Kondensatoren C5 und C6 wurde und unter Zusatz von 4-Methoxyphenol (MEHQ, 2% in TBMA)-Lösung als Stabilisator I6 als Rücklauf in die Destillationskolonne D2 geleitet. Weitere Teilströme der vereinigten Kondensate P1 und P2 aus den Kondensatoren C5 und C6 wurden und unter Zusatz von 4-Methoxyphenol (MEHQ, 2% in TBMA)-Lösung als Stabilisatoren I7 und I8 den Kondensatoren C5 bzw. C6 zugeführt.

Hierbei wurde die Stabilisierung der Kolonne mit einem höheren Gehalt an MEHQ vorgenommen, während der 4-Methoxyphenol-Gehalt in den Kondensatoren C5 und C6 15 +/- 5 ppm betrug. Um die Kondensation von TBMA am Kolonnenkopf der Destillationskolonne D2 zu vermeiden, welche auch zur Polymerisation von TBMA führen könnte, wurde der Kolonnenkopf mit Dampf (4 bar abs.) beheizt.

Noch ein weiterer Teilstrom der vereinigten Kondensate P1 und P2 aus den Kondensatoren C5 und C6 wurde nach einer Druckerhöhung auf 4 bar abs. über einen Wärmetauscher (Spiral-Wärmetauscher, Kühlung: Kühlsole (-20 °C), in Fig.1 nicht dargestellt) auf 20 °C gekühlt und als Produkt aus dem Verfahren ausgetragen. Ein Teilstrom davon wurde als Lösungsmittel für die Stabilisatoren 4-HT und MEHQ verwendet.

Das Produkt hatte folgende Zusammensetzung:

| | |
|---|---|
| 99,68% | TBMA |
| 0,30% | Isobuten |
| 100 ppm | Säure (MAS, Essigsäure, Propionsäure) |
| 17 ppm | MEHQ |

Der Sumpf S2 der Destillationskolonne D2 (umfassend 91,35% MAS) wurde nach einer Druckerhöhung auf 4 bar abs. über einen Wärmetauscher (Spiral-Wärmetauscher, 5 m², Kühlung: Warmwasser Edelstahl 1.4571, in Fig.1 nicht dargestellt) auf 35°C gekühlt und ein Teilstrom mit dem Zufuhrstrom der Methacrylsäure vereinigt und dem Reaktor R1 zugeführt.

Der in der Verdampfungseinheit V1 und den nachgeschalteten Einheiten benötigte Unterdruck wurde mittels einer Vakuumeinheit erzeugt. Es wurden Drehwälzkolbenverdichter ohne Schmieröl verwendet.

Zur Herstellung der Stabilisatorlösung von Phenothiazin wurde Methacrylsäure rein in einem Rührbehälter (begleitbeheizt mit Wasser, 30 °C, belüftet) vorgelegt. PTZ wurde als Feststoff über eine Sackentleerstation und ein pneumatisches Powder-Transfer-System mittels Anlegen von Vakuum über eine Pumpe in den Rührbehälter eingeführt. Geringe Mengen an Magerluft (5 Vol.-% Sauerstoff in Stickstoff) wurden zugegeben. PTZ wurde unter Rühren gelöst und die PTZ-Lösung in einen Vorlagebehälter (begleitbeheizt mit Wasser, 30 °C, belüftet) geleitet, von diesem aus die Dosierung ins Verfahren vorgenommen wurde.

Zur Herstellung der Stabilisatorlösung von 4-Hydroxy-2,2,6,6-tetramethyl-1-oxyl-piperidin wurde tert-Butylmethacrylat (aus den vereinigten Kondensaten P1 und P2 der Kondensatoren C5 und C6) in einem Rührbehälter (belüftet) vorgelegt. 4-HT wurde als Feststoff über eine Sackentleerstation und ein pneumatisches Powder-Transfer-System mittels Anlegen von Vakuum über eine Pumpe in den Rührbehälter eingeführt. Geringe Mengen an Magerluft (5 Vol.-% Sauerstoff in Stickstoff) wurden zugegeben. 4-HT wurde unter Rühren gelöst und die 4-HT-Lösung in einen Vorlagebehälter (belüftet) geleitet, von diesem aus die Dosierung ins Verfahren vorgenommen wurde.

Zur Herstellung der Stabilisatorlösung von 4-Methoxyphenol wurde tert-Butylmethacrylat (aus den vereinigten Kondensaten P1 und P2 der Kondensatoren C5 und C6) in einem Rührbehälter (belüftet) vorgelegt. MEHQ wurde als Feststoff über eine Sackentleerstation und ein pneumatisches Powder-Transfer-System mittels Anlegen von Vakuum über eine Pumpe in den Rührbehälter eingeführt. Geringe Mengen an Magerluft (5 Vol.-% Sauerstoff in Stickstoff) wurden zugegeben. MEHQ wurde unter Rühren gelöst und die MEHQ-Lösung in einen Vorlagebehälter (belüftet) geleitet, von diesem aus die Dosierung ins Verfahren vorgenommen wurde.

Die in den Solekühlern verwendet Sole war als Druckkreis aufgebaut. Die Sole wurde in einer Ammoniakkälteanlage auf -20 °C gekühlt und den jeweiligen Verfahrenselementen zugeführt. Danach wurde die Sole in einem Solevorlagebehälter homogenisiert und über eine Pumpe wieder der Ammoniakkälteanlage zugeführt. Das Solesystem verfügte über ein Ausgleichsgefäß, welches mit Magerluft (5 Vol.-% Sauerstoff in Stickstoff) überlagert wurde.

Beim Verfahren anfallendes, nicht verwertbares Abgas wurde über einen Abscheider geführt und die nicht kondensierten Bestandteile an einer Schirmfackel verbrannt, während das Kondensat ausgeführt wurde.

Es ist erkennbar, dass das Verfahren die Herstellung von tert-Butylmethacrylsäureester in hoher Reinheit (hier 99,68%) bei gleichzeitig energetisch günstiger Abtrennung von Isobuten, welches mit hohem Abtrennungsgrad aus dem Veresterungsgemisch isoliert werden konnte, erlaubt.

### Beispiel 1 - Variante 1 (Vergleichsbeispiel)

Das Verfahren wurde gemäß Beispiel 1 durchgeführt. Die Reindestillation wurde mit einem Naturumlaufverdampfer (4 bar abs. Dampf, 114 Rohre) und ohne zusätzliche Wandbeheizung im Kopfbereich betrieben.

| | |
|---|---|
| Temperatur im Sumpf der Reindestillation: | ca. 102 °C |
| Temperatur im Kopf der Reindestillation: | ca. 58 °C |
| Wandtemperatur im Kopfbereich: | ca. 53 °C |
| Rücklaufverhältnis: | 3,1 |
| Heizdampf zum Naturumlaufverdampfer: | ca. 400 kg/h |
| Differenzdruck in der Kolonne: | ca. 55 mbar |

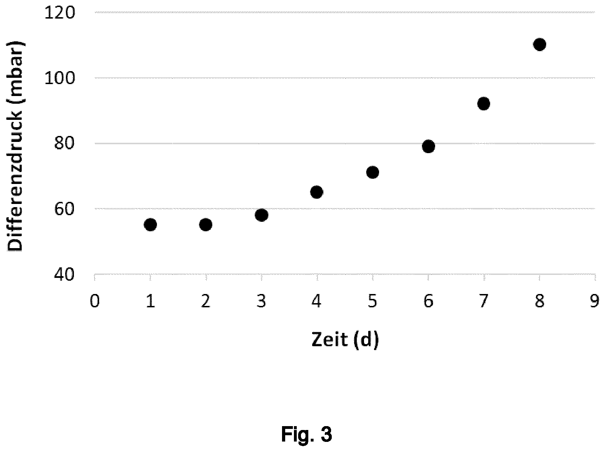

Es wurde der Differenzdruck in der Reinkolonne D2 in Abhängigkeit von der Zeit untersucht. Der Differenzdruck und die Heizleistung gelten als Indikatoren für den Polymerisationsgrad in der Kolonne. Es wurde ein Anstieg des Differenzdruckes in der Reinkolonne D2 von etwa 55 mbar auf etwa 110 mbar über einen Zeitraum von etwa 8 Tagen beobachtet (Fig. 3). Der Druckanstieg wurde durch die Kondensation gasförmiger Produkte an den Oberflächen im Kopfbereich der Kolonne verursacht, welche nicht stabilisierte polymerisationsanfällige Flüssigphasen bildeten und zu einem Belag auf den Oberflächen im Kopfbereich der Kolonne führte.

Zudem wurde eine abnehmende Heizleistung (Wärmeübergang) des Naturumlaufverdampfers festgestellt. Die benötigte Heizdampfmenge erhöhte sich von ca. 400 kg/h auf ca. 500 kg/h.

Die oberen zwei Böden der Kolonne wurden ausgebaut und mussten gereinigt werden, da sie mit Polymerbrocken belegt waren. Ca. 30% der inneren Verdampferrohre waren mit Polymer verstopft und mussten ebenfalls aufwendig gereinigt werden.

### Beispiel 1 - Variante 2 (Vergleichsbeispiel)

Das Verfahren wurde gemäß Beispiel 1 durchgeführt. Die Reindestillation wurde mit einem Naturumlaufverdampfer (4 bar abs. Dampf, 114 Rohre) und mit einer Wandbeheizung im Kopfbereich mittels Ummantelung mit einer Halbrohrschlange (110 °C, 1,5 bar) betrieben.

| | |
|---|---|
| Temperatur im Sumpf der Reindestillation: | ca. 102 °C |
| Temperatur im Kopf der Reindestillation: | ca. 58 °C |
| Wandtemperatur im Kopfbereich: | ca. 66 °C |
| Rücklaufverhältnis: | 3,1 |
| Heizdampf zum Naturumlaufverdampfer: | ca. 400 kg/h |
| Differenzdruck in der Kolonne: | ca. 55 mbar |

Es wurde die Heizleistung des Naturumlaufverdampfers in Abhängigkeit von der Zeit untersucht. Die Heizleistung gilt als Indikator für den Polymerisationsgrad in der Kolonne. Es wurde eine abnehmende Heizleistung (Wärmeübergang) des Naturumlaufverdampfers festgestellt. Die benötigte Heizdampfmenge erhöhte sich von ca. 400 kg/h auf ca. 500 kg/h.

Das Verfahren musste nach 15 Tagen unterbrochen werden. Der Kolonnenkopf wurde geöffnet und es konnten weder im Kolonnenkopf, noch auf dem obersten Boden ein Polymerbelag festgestellt werden. Ca. 30% der inneren Verdampferrohre waren allerdings mit Polymer verstopft und mussten aufwendig gereinigt werden

### Beispiel 1 - Variante 3

Das Verfahren wurde gemäß Beispiel 1 durchgeführt. Die Reindestillation wurde mit einem Zwangsumlaufentspannungsverdampfer (4 bar abs. Dampf, 308 Rohre) und mit einer Wandbeheizung im Kopfbereich mittels Ummantelung mit einer Halbrohrschlange (110 °C, 1,5 bar) betrieben.

| | |
|---|---|
| Temperatur im Sumpf der Reindestillation: | ca.102 °C |
| Temperatur im Kopf der Reindestillation: | ca. 58 °C |
| Wandtemperatur im Kopfbereich: | ca. 66 °C |
| Rücklaufverhältnis: | 3,1 |
| Heizdampf zum Verdampfer mit Zwangsumlauf: | ca. 400 kg/h |
| Differenzdruck in der Kolonne: | ca. 55 mbar |

Es wurden der Differenzdruck in der Reinkolonne D2 sowie die Heizleistung des Zwangsumlaufverdampfers in Abhängigkeit von der Zeit untersucht. Der Differenzdruck und die Heizleistung gelten als Indikatoren für den Polymerisationsgrad in der Kolonne. Es wurde über einen Zeitraum von 30 Tagen kein signifikanter Anstieg des Differenzdruckes in der Reinkolonne beobachtet.

Das Verfahren wurde unterbrochen. Der Kolonnenkopf wurde geöffnet und es konnte weder im Kolonnenkopf, noch auf dem obersten Boden oder in den Zwangsumlaufverdampferrohren ein Polymerbelag festgestellt werden.

## Patentansprüche

1. Verfahren zur kontinuierlichen Herstellung des tert-Butylesters einer ethylenisch ungesättigten Carbonsäure, bei dem man
a) eine ethylenisch ungesättigte Carbonsäure mit Isobuten in Gegenwart eines sauren Katalysators zu einem Veresterungsgemisch umsetzt;
b) den sauren Katalysator abtrennt;
c) leichtsiedende Komponenten abtrennt; und
d) eine tert-Butylester enthaltende Flüssigkeit einer Destillationsvorrichtung zuführt und in der Destillationsvorrichtung reindestilliert, wobei man
d1) die tert-Butylester enthaltende Flüssigkeit in der Destillationsvorrichtung in ein tert-Butylester enthaltendes gasförmiges Kopfprodukt und ein Carbonsäure enthaltendes flüssiges Sumpfprodukt auftrennt;
d2) das tert-Butylester enthaltende gasförmige Kopfprodukt zumindest teilweise kondensiert und das Kondensat teilweise als Rücklauf in die Destillationsvorrichtung zurückführt;
d3) das Carbonsäure enthaltende flüssige Sumpfprodukt zumindest teilweise in Schritt a) zurückführt;
d4) Carbonsäure enthaltendes flüssiges Sumpfprodukt abzieht und zu einem Aufheizer leitet, einen überhitzten, flüssigen Rückführstrom aus dem Aufheizer entnimmt und den überhitzten Rückführstrom in die Destillationsvorrichtung entspannt; und
d5) zumindest im Kopfbereich der Destillationsvorrichtung die mit dem Brüden in Kontakt stehenden Wände der Destillationsvorrichtung zumindest in Teilbereichen beheizt und/oder thermisch isoliert.

2. Verfahren nach Anspruch 1, wobei die ethylenisch ungesättigte Carbonsäure ausgewählt ist unter Acrylsäure und Methacrylsäure.

3. Verfahren nach einem der vorhergehenden Ansprüche, wobei die ethylenisch ungesättigte Carbonsäure einen Essigsäuregehalt von weniger als 300 ppm und einen Propionsäuregehalt von weniger als 300 ppm aufweist.

4. Verfahren nach einem der vorhergehenden Ansprüche, wobei man
d1') das flüssige Sumpfprodukt über eine Vorrichtung führt, welche es von festen Verunreinigungen befreit.

5. Verfahren nach Anspruch 4, wobei die Vorrichtung ein Filter ist.

6. Verfahren nach einem der vorhergehenden Ansprüche, wobei der Aufheizer ein in indirektem Wärmetausch gegen ein Heizmedium arbeitender Rohrbündel-Wärmetauscher ist.

7. Verfahren nach einem der vorhergehenden Ansprüche, wobei die Entspannung des überhitzten Rückführstroms über ein Durchflussbegrenzungsmittel erfolgt, ausgewählt unter einer Blende, einem Ventil, einer Drossel, einer Lochscheibe, einer Düse, einer Kapillare oder Kombinationen davon.

8. Verfahren nach einem der vorhergehenden Ansprüche, wobei der saure Katalysator eine anorganische Säure ist.

9. Verfahren nach einem der Ansprüche 1 bis 7, wobei der saure Katalysator eine organische Säure ist.

10. Verfahren nach einem der vorhergehenden Ansprüche, wobei das Veresterungsgemisch 0,1 bis 10 Gew.-% des sauren Katalysators umfasst.

11. Verfahren nach einem der vorhergehenden Ansprüche, wobei die Umsetzung in Schritt a) in Gegenwart eines unter Phenothiazinen ausgewählten Stabilisators durchgeführt wird.

12. Verfahren nach einem der vorhergehenden Ansprüche, wobei in den Verstärkungsteil der Destillationsvorrichtung ein unter Phenolverbindungen ausgewählter Stabilisator dosiert wird.

13. Verfahren nach einem der vorhergehenden Ansprüche, wobei in den Zulauf zur Destillationsvorrichtung ein unter N-Oxyl-Verbindungen ausgewählter Stabilisator dosiert wird.

14. Verfahren nach einem der vorhergehenden Ansprüche, wobei das Volumenverhältnis von Sauerstoff zu Stickstoff in allen gasförmigen Gemischen, die in den Stufen a) bis d) auftreten, im Bereich von 0,03 bis 0,11 liegt.

## Claims

1. A process for continuously preparing the tert-butyl ester of an ethylenically unsaturated carboxylic acid, by
a) reacting an ethylenically unsaturated carboxylic acid with isobutene in the presence of an acidic catalyst to give an esterification mixture;
b) removing the acidic catalyst;
c) removing low-boiling components; and
d) supplying a tert-butyl ester-comprising liquid to a distillation apparatus and subjecting it to purifying distillation in the distillation apparatus, where
d1) in the distillation apparatus the tert-butyl ester-comprising liquid is separated into a tert-butyl ester-comprising gaseous top product and a carboxylic acid-comprising liquid bottom product;
d2) the tert-butyl ester-comprising gaseous top product is at least partly condensed and the condensate is recycled partly as reflux to the distillation apparatus;
d3) the carboxylic acid-comprising liquid bottom product is recycled at least partly to step a) ;
d4) carboxylic acid-comprising liquid bottom product is drawn off and passed to a heater; a superheated, liquid recycle stream is taken from the heater; and the superheated recycle stream is let down into the distillatiuon apparatus; and
d5) at least in the top region of the distillation apparatus, the distillation apparatus walls in contact with the vapor, at least in sub-regions, are heated and/or thermally insulated.

2. The process according to claim 1, the ethylenically unsaturated carboxylic acid being selected from acrylic acid and methacrylic acid.

3. The process according to either of the preceding claims, the ethylenically unsaturated carboxylic acid having an acetic acid content of less than 300 ppm and a propionic acid content of less than 300 ppm.

4. The process according to any of the preceding claims, where
d1') the liquid bottom product is guided via an apparatus which frees it from solid impurities.

5. The process according to claim 4, the apparatus being a filter.

6. The process according to any of the preceding claims, the heater being a shell and tube heat exchanger which operates in indirect heat exchange against a heating medium.

7. The process according to any of the preceding claims, the superheated recycle stream being let down via a flow limiter selected from a baffle, a valve, a constrictor, a perforated plate, a nozzle, a capillary, or combinations thereof.

8. The process according to any of the preceding claims, the acidic catalyst being an inorganic acid.

9. The process according to any of claims 1 to 7, the acidic catalyst being an organic acid.

10. The process according to any of the preceding claims, the esterification mixture comprising 0.1 to 10 wt% of the acidic catalyst.

11. The process according to any of the preceding claims, the reaction in step a) being carried out in the presence of a stabilizer selected from phenothiazines.

12. The process according to any of the preceding claims, a stabilizer selected from phenol compounds being metered into the rectifying section of the distillation apparatus.

13. The process according to any of the preceding claims, a stabilizer selected from N-oxyl compounds being metered into the feed to the distillation apparatus.

14. The process according to any of the preceding claims, the volume ratio of oxygen to nitrogen in all gaseous mixtures which occur in stages a) to d) being in the range from 0.03 to 0.11.

## Revendications

1. Procédé de fabrication continue de l'ester tert-butylique d'un acide carboxylique éthyléniquement insaturé, selon lequel
a) un acide carboxylique éthyléniquement insaturé est mis en réaction avec de l'isobutène en présence d'un catalyseur acide pour former un mélange d'estérification ;
b) le catalyseur acide est séparé ;
c) les composants de point d'ébullition faible sont séparés ; et
d) un liquide contenant l'ester tert-butylique est introduit dans un dispositif de distillation et soumis à une distillation pure dans le dispositif de distillation,
d1) le liquide contenant l'ester tert-butylique étant séparé dans le dispositif de distillation en un produit de tête gazeux contenant l'ester tert-butylique et un produit de fond liquide contenant l'acide carboxylique ;
d2) le produit de tête gazeux contenant l'ester tert-butylique étant au moins partiellement condensé, et le condensat étant partiellement recyclé en tant que reflux dans le dispositif de distillation ;
d3) le produit de fond liquide contenant l'acide carboxylique étant au moins partiellement recyclé dans l'étape a) ;
d4) le produit de fond liquide contenant l'acide carboxylique étant soutiré et acheminé dans un dispositif de chauffage, un courant de recyclage liquide surchauffé étant extrait du dispositif de chauffage, et le courant de recyclage surchauffé étant détendu dans le dispositif de distillation ; et
d5) au moins dans la zone de tête du dispositif de distillation, les parois du dispositif de distillation en contact avec les vapeurs étant chauffées et/ou isolées thermiquement au moins dans des zones partielles.

2. Procédé selon la revendication 1, dans lequel l'acide carboxylique éthyléniquement insaturé est choisi parmi l'acide acrylique et l'acide méthacrylique.

3. Procédé selon l'une quelconque des revendications précédentes, dans lequel l'acide carboxylique éthyléniquement insaturé présente une teneur en acide acétique de moins de 300 ppm et une teneur en acide propionique de moins de 300 ppm.

4. Procédé selon l'une quelconque des revendications précédentes, dans lequel
d1') le produit de fond liquide est passé par un dispositif qui le débarrasse des impuretés solides.

5. Procédé selon la revendication 4, dans lequel le dispositif est un filtre.

6. Procédé selon l'une quelconque des revendications précédentes, dans lequel le dispositif de chauffage est un échangeur de chaleur à faisceau de tubes qui fonctionne en échange de chaleur indirect avec un milieu chauffant.

7. Procédé selon l'une quelconque des revendications précédentes, dans lequel la détente du courant de recyclage surchauffé a lieu par un moyen de limitation du débit, choisi parmi un diaphragme, une soupape, un étrangleur, un disque perforé, une buse, un capillaire ou des combinaisons de ceux-ci.

8. Procédé selon l'une quelconque des revendications précédentes, dans lequel le catalyseur acide est un acide inorganique.

9. Procédé selon l'une quelconque des revendications 1 à 7, dans lequel le catalyseur acide est un acide organique.

10. Procédé selon l'une quelconque des revendications précédentes, dans lequel le mélange d'estérification comprend 0,1 à 10 % en poids du catalyseur acide.

11. Procédé selon l'une quelconque des revendications précédentes, dans lequel la réaction à l'étape a) est réalisée en présence d'un stabilisateur choisi parmi les phénothiazines.

12. Procédé selon l'une quelconque des revendications précédentes, dans lequel un stabilisateur choisi parmi les composés de phénol est ajouté dans la zone d'enrichissement du dispositif de distillation.

13. Procédé selon l'une quelconque des revendications précédentes, dans lequel un stabilisateur choisi parmi les composés de N-oxyle est ajouté dans l'alimentation du dispositif de distillation.

14. Procédé selon l'une quelconque des revendications précédentes, dans lequel le rapport volumique entre l'oxygène et l'azote dans tous les mélanges gazeux qui se forment dans les étapes a) à d) se situe dans la plage allant de 0,03 à 0,11.
